(19) Europäisches Patentamt
European Patent Office
Office européen des brevets

(11) **EP 4 150 315 B1**

(12) **FASCICULE DE BREVET EUROPEEN**

(45) Date de publication et mention de la délivrance du brevet:
**08.07.2026 Bulletin 2026/28**

(21) Numéro de dépôt: **21731251.1**

(22) Date de dépôt: **11.05.2021**

(51) Classification Internationale des Brevets (IPC):
**G01N 7/14** *(2006.01)* **G01N 7/22** *(2006.01)*
**G01N 33/02** *(2006.01)* **G01N 33/42** *(2006.01)*
**G01N 33/44** *(2006.01)*

(52) Classification Coopérative des Brevets (CPC):
**G01N 7/14; G01N 7/22**

(86) Numéro de dépôt international:
**PCT/FR2021/050816**

(87) Numéro de publication internationale:
**WO 2021/229176 (18.11.2021 Gazette 2021/46)**

(54) **DISPOSITIF DE MESURE DE PROPRIETES PHYSICO-CHIMIQUES D'UNE MATRICE DEFORMABLE, PROCEDE DE MISE EN OEUVRE ET UTILISATIONS**

VORRICHTUNG ZUR MESSUNG PHYSIKALISCH-CHEMISCHER EIGENSCHAFTEN EINER VERFORMBAREN MATRIX, IMPLEMENTIERUNGSVERFAHREN UND VERWENDUNGEN

DEVICE FOR MEASURING PHYSICOCHEMICAL PROPERTIES OF A DEFORMABLE MATRIX, IMPLEMENTATION METHOD AND USES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.05.2020 FR 2004751**

(43) Date de publication de la demande:
**22.03.2023 Bulletin 2023/12**

(73) Titulaire: **Institut national de recherche pour l'agriculture, l'alimentation et l'environnement 75007 Paris (FR)**

(72) Inventeurs:
• GRENIER, David
**35340 ERCÉ-PRÈS-LIFFRÉ (FR)**
• LEJEUNE, Antoine
**53950 LOUVERNÉ (FR)**
• LUCAS, Tiphaine
**35490 SENS-DE-BRETAGNE (FR)**

• **DIASCORN, Yves
35590 L'HERMITAGE (FR)**

(74) Mandataire: **Novagraaf Technologies 2 rue Sarah Bernhardt CS90017 92665 Asnières-sur-Seine Cedex (FR)**

(56) Documents cités:
**EP-A1- 0 422 260         EP-A1- 0 687 900
EP-B1- 0 422 260         US-A- 2 233 372
US-A1- 2016 327 456**

• **RZIGUE ASMA ET AL: "Bread collapse. Causes of the technological defect and impact of depanning time on bread quality", JOURNAL OF FOOD ENGINEERING, BARKING ESSEX, GB, vol. 182, 17 March 2016 (2016-03-17), pages 72 - 80, XP029488952, ISSN: 0260-8774, DOI: 10.1016/ J.JFOODENG.2016.03.007**

**Description**

**Domaine technique**

La présente invention se rapporte à un dispositif de mesure de propriétés physico-chimiques d'un matériau vis-à-vis de gaz, un procédé de mesure de la pression d'un gaz au contact d'un matériau, à l'aide du dispositif de mesure ainsi qu'à l'utilisation du dispositif pour la mesure, dans un matériau, d'au moins une propriété physico-chimique vis-à-vis de gaz choisies parmi des propriétés de transport de matière et des propriétés mécaniques.

La présente invention trouve une application notamment dans les domaines agroalimentaires et du batîment.

Dans la description ci-dessous, les références entre crochets ([]) renvoient à la liste des références présentée à la fin du texte.

**Etat de la technique**

La connaissance des propriétés physico-chimiques des matrices déformables vis-à-vis de gaz, en particulier le dioxyde de carbone ($CO_2$), est importante pour optimiser leurs procédés de production et assurer la qualité du produit final.

Quel que soit le domaine technique auquel appartient ces matrices déformables, la détermination de ces propriétés reste à ce jour longue et coûteuse, car elle nécessite de mettre en place des séries d'expériences spécifiques et indépendantes pour chacune d'entre elles, impliquant diverses méthodes analytiques, comme les dosages chimiques (Acerbi et al.: "Impact of salt concentration, ripening temperature and ripening time on CO2 production of semi-hard cheese with propionic acid fermentation", Journal of Food Engineering (2016) ([2]) ; Jakobsen M., Nygaard Jensen P. : « Assessment of carbon dioxide solubility coefficients for semi-hard cheeses : the effect of temperature and fat content », Eur. Food Res. Technol., 229, 287-294 (2009) ([8])) ou électrochimiques (Chaix et al.: "Oxygen and carbon dioxide solubility and diffusivity in solid food matrix: a review of past and current knowledge", Comprehensive reviews in food science and food safety (2014) ([7])), les mesures par spectroscopie infrarouge (Chaix et al ([7])) ou encore par chromatographie (Chaix et al ([7])). Ces propriétés peuvent en plus évoluer avec le temps, rendant leur mesure encore plus fastidieuse. En outre, elles permettent difficilement d'effectuer des mesures « à cœur » (in situ), puisqu'elles requièrent le prélèvement d'un ou plusieurs bouts de la matrice alimentaire, entraînant une incertitude de mesure due au contact de la matrice alimentaire avec l'atmosphère ambiant. Dans le cas des matrices alimentaires, comme les pains, les viennoiseries ou les fromages, les micro-organismes de la matrice alimentaire sont alors exposés à l'oxygène de l'air, et les gaz initialement solubilisés dans la matrice se dé-solubilisent quasi instantanément en surface, modifiant alors les paramètres mesurés par rapport à ce qu'ils étaient in situ.

Le document EP 0 422 260 décrit un dispositif de mesure de propriétés physico-chimiques vis-à-vis de gaz au contact d'un matériau, comprenant une extrémité supérieure dans laquelle est inséré hermétiquement un capteur de pression relié à un appareil d'enregistrement et éventuellement de traitement d'un signal, et une extrémité inférieure qui est en communication avec ledit capteur de pression. Toutefois, ce dispositif ne permet pas d'effectuer une mesure de certains paramètres physico-chimiques comme les propriétés de transport, notamment le coefficient de diffusion, la constante d'équilibre gaz gazeux/gaz dissout, la concentration en gaz dissous et la vitesse de production, et des propriétés mécaniques comme l'élasticité, la viscosité, la visco-élasticité et le point de fracture.

Ainsi, il n'existe actuellement pas d'outil capable de mesurer rapidement, notamment dans des matrices alimentaires, différents paramètres physico-chimiques de ces matrices vis-à-vis de gaz.

Il existe donc un réel besoin de dispositifs et procédés permettant de mesurer différentes propriétés physico-chimiques d'une matrice déformable palliant ces défauts, inconvénients et obstacles de l'art antérieur.

**Description de l'invention**

Les inventeurs sont parvenus à répondre à ces besoins par la mise au point d'un dispositif de mesure permettant de s'affranchir des difficultés citées précédemment, dont le principe de fonctionnement, original, repose sur la mesure des variations de pressions dans une phase gazeuse au contact d'une matrice, notamment alimentaire, suite à diverses sollicitations en pressions, pendant des durées allant de quelques minutes à quelques heures, voire quelques jours, selon les paramètres à mesurer. Ainsi, avantageusement, un seul type de mesure est nécessaire pour déterminer toutes les propriétés physico-chimiques désirées.

Le principe de fonctionnement de la sonde est de mesurer des variations de pressions dans une phase gazeuse au contact de la matrice à analyser suite à diverses sollicitations en pression pendant des durées allant de quelques minutes à quelques heures, éventuellement quelques jours, de préférence sans excéder 4 jours, selon les paramètres à mesurer. Ainsi, un seul type de mesure est nécessaire pour déterminer toutes les propriétés physico-chimiques désirées. L'originalité de la sonde repose sur la mise en place d'un système de balayage en gaz afin de s'assurer que le milieu soit uniquement composé de ce gaz. Par ailleurs, la sonde permet de faire des mesures à cœur.

De plus, les caractéristiques techniques du dispositif de l'invention permettent des mesures plus rapides et à cœur (in situ), ce qui est une amélioration par rapport aux méthodes utilisées classiquement qui nécessitent de prélever un morceau de la matrice et de la mettre en contact avec l'atmosphère ambiant, ce qui peut modifier ses propriétés. Les résultats obtenus par la mise en œuvre du dipositif de l'invention sont donc plus pertinents que ceux mesurés après prélèvements de morceaux de matrice.

En outre, la simplicité d'utilisation du dispositif de l'invention, sa compacité et sa rapidité de mesure sont autant d'avantages pour les industriels. Elles permettent notamment l'évaluation des propriétés sur site, par exemple sur des meules en cours d'affinage, sur une pâte à pain en cours de fermentation ou encore pendant la fabrication d'une dalle de ciment.

Par ailleurs, le fait d'utiliser un seul appareil pour mesurer différentes, voire toutes les propriétés physico-chimiques, permet de s'affranchir de l'achat coûteux d'autres outils et d'une formation spécifique sur chacun de ces outils pour l'utilisateur.

Toutes ces propriétés sont en outre utilisables pour le contrôle des procédés de production et aussi dans les centres de recherche et développement. Elles servent toutes à la compréhension et à la modélisation de la croissance de bulles, par exemple dans le fromage où le nombre, la distribution et la taille des bulles sont des critères de choix primordiaux chez les consommateurs. Enfin, la solubilité et la diffusion des gaz, notamment du $CO_2$, sont utiles pour choisir les conditionnements des aliments.

Ainsi, un premier objet de l'invention se rapporte à un dispositif de mesure de propriétés physico-chimiques vis-à-vis de gaz au contact d'un matériau, comprenant :

- une extrémité supérieure, dans laquelle est inséré hermétiquement un capteur de pression relié à un appareil d'enregistrement et éventuellement de traitement d'un signal,
- une extrémité inférieure, qui est en communication avec ledit capteur de pression et qui est ouverte pour permettre l'insertion du dispositif de mesure dans le matériau et la formation d'une chambre gazeuse entre ledit capteur de pression et le matériau lorsque ledit dispositif de mesure est inséré dans celui-ci,

caractérisé en ce que ledit dispositif comprend en outre :

- un système de balayage en un gaz,
- au moins un moyen d'entrée dudit gaz dans le dispositif, ledit moyen étant relié à une source externe de gaz et n'étant pas un moyen de prélèvement du gaz inclus dans le matériau,, et au moins un moyen de sortie dudit gaz hors du dispositif, ledit dispositif étant en une matière n'absorbant pas ledit gaz.

On entend par « matériau », au sens de la présente invention, tout matériau dans lequel au moins un gaz est susceptible de se solubiliser et de diffuser. Ainsi, le matériau peut être un matériau déformable. Le matériau peut être alvéolaire ou non alvéolaire. En outre, il peut s'agir d'une matrice alimentaire, ou d'une matrice non alimentaire. Dans le cas d'une matrice alimentaire, il peut s'agir par exemple d'une matrice choisie parmi un produit fromager, un produit de boulangerie, comme une pâte à pain, une viande, un poisson, un produit à base de viande ou de poisson, un fruit, un légume, un produit à base de fruit ou de légume, une pâte alimentaire, et leurs mélanges. Dans le cas d'une matrice non alimentaire, il peut s'agir par exemple d'une matrice choisie parmi du bitume, du béton, du ciment, de l'asphalte, du plâtre, des polymères, des gels, de la terre, du bois, du silicone, du charbon, des roches, et leurs mélanges.

On entend par « propriétés physico-chimiques », au sens de la présente invention, toute propriété de transport de matière vis-à-vis de gaz, ainsi que les propriétés mécaniques. Avantageusement, les propriétés de transport de matière peuvent être choisies parmi le coefficient de diffusion, la constante d'équilibre gaz gazeux/gaz dissout, la concentration en gaz dissous et la vitesse de production. Les propriétés mécaniques peuvent être choisies parmi l'élasticité, la viscosité, la visco-élasticité et le point de fracture.

De manière avantageuse, le gaz vis-à-vis duquel on veut déterminer les propriétés physico-chimique du matériau peut être tout gaz susceptible de se solubiliser et de diffuser dans le matériau. Il peut s'agir par exemple d'un gaz produit par le matériau lui-même, par exemple dans le cas d'une matrice alimentaire fermentescible. Il peut notamment s'agir d'un gaz choisi parmi le dioxyde de carbone, l'azote, l'oxygène, les gaz rares, les composés organiques volatiles, l'ammoniac, et un mélange de ceux-ci.

Le dispositif de l'invention est réalisé en une matière n'absorbant pas ledit gaz vis-à-vis duquel on veut déterminer les propriétés physico-chimique du matériau. Cela est en effet nécessaire pour ne pas biaiser les mesures. Il peut s'agir d'une matière choisie parmi le métal, le verre et des matériaux polymères préalablement saturés en ledit gaz ou traités pour ne pas absorber ce gaz. En tout état de cause, les matières plastiques non préalablement saturées en ledit gaz ou non préalablement traitées pour ne pas absorber ce gaz sont proscrites car elles absorbent certains gaz, comme le $CO_2$.

La forme du dispositif peut être toute forme adaptée en fonction de l'utilisation souhaitée et du matériau concerné. A cet égard, l'homme du métier saura adapter cette caractéristique au vu de ses connaissances générales. Il peut s'agir par exemple d'un tube creux, de forme pouvant être cylindrique, ovale ou polygonale, par exemple carrée, rectangulaire ou hexagonale. De préférence, le dispositif est un tube creux, éventuellement cylindrique.

La taille du dispositif peut être choisie en fonction de l'utilisation souhaitée et du matériau concerné. A cet égard, l'homme du métier saura adapter cette caractéristique au vu de ses connaissances générales. Par exemple, le dispositif peut être un dispositif portatif ou un dispositif fixe. Avantageusement :

La hauteur du dispositif peut être au minimum de quelques millimètres, voire quelques dizaines de millimètres, afin de pouvoir facilement insérer la sonde « à cœur » dans le matériau. La hauteur du dispositif peut ainsi être supérieure ou égale à 5 mm, par exemple d'environ 10 mm, ou environ 20 mm, ou environ 30 mm, ou environ 40 mm, ou environ 50 mm, voire supérieure à 50 mm, selon l'usage qui en est fait.

La hauteur entre l'extrémité inférieure du dispositif et le capteur de pression est avantageusement supérieure ou égale à 1 mm de manière à pouvoir facilement « planter » le dispositif dans le matériau sur une hauteur minimale de 1 mm pour respecter certaines hypothèses de calcul pour le traitement des données. Par exemple, cette hauteur peut être comprise entre 1 et 8 mm, voire supérieure à 8 mm comme indiqué précédemment.

Le diamètre du dispositif peut être le même sur toute la longueur du dispositif, ou substantiellement le même. Il est préférentiellement de l'ordre de quelques millimètres, par exemple entre 2 et 10 mm, pour acquérir rapidement les données voulues. Eventuellement, le diamètre peut être supérieur à 10 mm. Avantageusement, le diamètre du dispositif peut être choisi en fonction de la durée souhaitée d'une analyse ; en effet, d'une manière générale, plus le volume de la chambre gazeuse est petit et plus certaines analyses peuvent être rapides.

Avantageusement, la hauteur du dispositif peut être supérieure ou égale à 5 mm, et la hauteur entre l'extrémité inférieure du dispositif et le capteur de pression est supérieure à 1 mm.

On entend par « extrémité inférieure », au sens de la présente invention, l'extrémité du dispositif qui a pour vocation d'être en contact avec le matériau. Elle est par ailleurs ouverte pour permettre au dispositif d'être enfoncé dans le matériau.

On entend par « extrémité supérieure », au sens de la présente invention, l'extrémité du dispositif qui est à l'opposé de l'extrémité inférieure, et qui n'a pas vocation à être en contact avec le matériau. Ainsi, avantageusement, seule l'extrémité inférieure du dispositif de l'invention est insérée dans le matériau, et non l'extrémité supérieure. Comme indiqué précédemment, le dispositif comprend un capteur de pression inséré hermétiquement dans l'extrémité supérieure du dispositif, de manière à maîtriser la pression dans la chambre gazeuse formée entre le capteur de pression et le matériau lorsque le dispositif de mesure est inséré dans celui-ci. Ainsi, il n'y a pas d'échanges gazeux non désiré entre la chambre gazeuse et l'air ambient, extérieur au dispositif. A cet effet, l'étanchéité vis-à-vis des gaz peut être assurée par tout moyen connu, par exemple une soudure entre le capteur de pression et les parois du dispositif.

Le capteur de pression peut être tout capteur disponible dans le commerce permettant de mesurer la force exercée par les gaz et susceptible d'être adapté au dispositif de l'invention. Il peut s'agir par exemple du capteur de pression Kulite Semiconductor Produtcs Inc, modèle XCQ-093-1.7.BARA.

L'appareil d'enregistrement auquel est relié le capteur de pression peut être tout appareil disponible dans le commerce du moment qu'il peut être adapté au capteur de pression utilisé. Avantageusement, mais de manière optionnelle, l'appareil d'enregistrement peut également traiter le signal reçu, c'est-à-dire corriger électroniquement les grandeurs d'erreur telles que le décalage, la sensibilité, l'effet de température sur le décalage, l'effet de la température sur la sensibilité, la non-linéarité et l'hystérésis. Avantageusement, l'appareil d'enregistrement peut traiter le signal afin de donner les valeurs numériques des propriétés physico-chimiques à déterminer. Alternativement, le traitement du signal peut être fait manuellement ou ultérieurement, dans le cas où le capteur n'a pas de fonction de traitement du signal.

L'insertion du dispositif de l'invention dans le matériau peut être réalisée en enfonçant l'extrémité inférieure du dispositif directement dans le matériau lorsque celui-ci le permet, ou bien dans un trou préalablement ménagé dans le matériau lorsque celui-ci est trop ferme. Ce trou peut être réalisé avec tout outil adapté, comme par exemple un foret. La profondeur du trou dépend du matériau considéré et sera déterminé par l'homme du métier au vu de ses connaissances techniques générales. Par exemple, le trou peut avoir une profondeur comprise entre 1 mm et 80 mm, notamment pour les matériaux du type pâte alimentaire comme le fromage. Une fois inséré dans le matériau, l'espace ménagé entre le matériau et l'extrémité supérieure, fermée hermétiquement, du dispositif, est appelé « chambre gazeuse ».

Avantageusement, le système de balayage en un gaz permet l'introduction, dans la chambre gazeuse, d'un gaz pour éliminer le gaz déjà présent, afin que le milieu de la chambre gazeuse soit uniquement composé de ce gaz. Le gaz utilisé à cet effet peut être tout gaz classiquement utilisé dans les systèmes de balayage en gaz des équipements industriels ou alimentaires, par exemple le $CO_2$, l'azote ou l'argon, ou encore le gaz vis-à-vis duquel on veut déterminer les propriétés de transport de matière. Avantageusement, pour les mesures des propriétés mécaniques, tout gaz peut être utilisé, et préférentiellement un gaz qui ne se solubilise pas ou que peu, comme le $CO_2$, l'azote ou l'argon. Pour les mesures des propriétés de tranport de matière, on utilise de préférence le gaz vis-à-vis duquel on veut déterminer les propriétés de transport de matière. Le système de balayage peut être un système disponible dans le commerce, par exemple une bouteille de gaz.

Le système de balayage en gaz est relié à au moins un moyen d'entrée en gaz. Ce moyen d'entrée peut être situé sur toute la hauteur du dispositif, par exemple soit dans la partie supérieure du dispositif, et/ou dans la partie inférieure du dispositif. Cette entrée peut être assurée par tout moyen classiquement utilisé à cet effet, comme par exemple un orifice. Avantageusement, l'entrée du gaz et éventuellement son débit peut être contrôlé par un dispositif adapté, par exemple une vanne, susceptible d'être ouverte ou fermée selon les besoins de l'utilisateur. Ainsi, comme il ressort des explications ci-avant, le moyen d'entrée du gaz est relié à une source externe de gaz, comme une bouteille de gaz, seringue ou une poche, cette liste n'étant pas limitative. En outre, selon l'invention, le moyen d'entrée du gaz n'est pas un moyen de prélèvement du gaz inclus dans le matériau. Le dispositif de l'invention comporte également au moins un moyen de sortie du gaz. Ce moyen de sortie peut être situé sur toute la hauteur du dispositif, par exemple soit dans la partie supérieure du dispositif, et/ou dans la partie inférieure du dispositif. Cette sortie peut être assurée par tout moyen classiquement utilisé à cet effet, comme par exemple un orifice. Avantageusement, la sortie du gaz et éventuellement son débit peut être contrôlé par un dispositif adapté, par exemple une vanne, susceptible d'être ouverte ou fermée selon les besoins de l'utilisateur.

Avantageusement, le dispositif de l'invention peut comprendre au moins un capteur de température permettant de mesurer la température dans la chambre gazeuse. Ce capteur peut être utile notamment lorsque la température est susceptible de varier pendant la mesure. A cet effet, tout capteur de température adapté et disponible dans le commerce peut être utilisé.

Dans un mode de réalisation compatible avec toutes les caractéristiques telles que définies précédemment, le dispositif de l'invention peut comprendre une extension reliée de manière étanche avec l'extrémité inférieure dudit dispositif. Avantageusement, l'extension peut être en une matière telle que définie ci-avant pour le dispositif. Il peut s'agir de la même matière que celle constituant le reste du dispositif, ou d'une autre matière telle que définie précédemment. L'extension peut être de forme similaire à celle du reste du dispositif, par exemple un tube creux, ouvert à son sommet pour communiquer avec le reste du dispositif. L'étanchéité entre le dispositif et l'extension peut être assurée par tout moyen connu de l'homme du métier, par exemple par un joint, un système de vissage ou une soudure. L'étanchéité entre l'extension et la matrice à analyser peut être assurée par tout moyen connu de l'homme du métier, par exemple un joint ou un système de vissage. Ce mode de réalisation est particulièrement utile pour la réalisation de mesures des propriétés mécaniques du matériau. L'extension doit disposer d'une aération ou de tout autre système permettant de laisser passer le gaz. Ainsi, l'extension peut avantageusement être percée sur au moins un côté et/ou percée en son extrémité inférieure, pour laisser passer le gaz.

Le dispositif de l'invention peut comprendre en outre au moins un moyen de maintien en position du dispositif par rapport audit matériau, par tout moyen adapté à cette fonction connu de l'homme du métier, comme par exemple des sangles, des pinces, ou des moyens d'attache à une potence.

Un autre objet de l'invention se rapporte à un procédé de mesure de la pression d'un gaz au contact d'un matériau, à l'aide d'un dispositif de mesure tel que défini précédemment, comprenant les étapes suivantes :

(a) insertion dudit dispositif de mesure dans le matériau,
(b) éventuellement balayage en un gaz de la chambre gazeuse au moyen du système de balayage en un gaz, à pression constante,
(c) variation de la pression de la chambre gazeuse au moyen du système de balayage en un gaz jusqu'à une pression désirée, et
(d) mesure de la pression et éventuellement de la température de la chambre gazeuse.

L'étape (a) insertion du dispositif peut se faire comme indiqué précédemment. Avantageusement, l'insertion peut être réalisée à une profondeur d'au moins 1 mm. En tout état de cause, l'homme du métier saura adapter la profondeur maximale en fonction de la hauteur entre l'extrémité inférieure du dispositif et le capteur de pression, et du matériau testé. La profondeur peut être par exemple comprise entre 1 et 10 mm.

L'étape (b), optionnelle, de balayage en un gaz de la chambre gazeuse au moyen du système de balayage en un gaz, est réalisée à pression constante, de manière à ce que la pression dans la chambre gazeuse n'évolue pas substantiellement. A cet effet, les moyens d'entrée et de sortie du gaz de balayage peuvent être ouverts. Le temps durant lequel cette étape est réalisée doit être suffisant pour permettre de remplacer l'air présent dans le dispositif par le gaz de balayage, et d'atteindre une pression constante dans la chambre gazeuse, et est donc fonction du volume de la chambre gazeuse. Cette étape permet également de régler la pression initiale à imposer pour réaliser les mesures. A titre d'exemple, cette durée peut être d'au moins 2 ou 3 secondes, de préférence sans dépasser quelques minutes. La pression peut être toute pression adaptée à la mesure à réaliser ; elle peut être par exemple comprise -1 kPa et +200 kPa (par rapport à la pression atmosphérique).

L'étape (c) de variation de la pression de la chambre gazeuse au moyen du système de balayage en un gaz jusqu'à une pression désirée peut être une augmentation ou une diminution de la pression de la chambre gazeuse. Cette étape est réalisée après l'étape (a) d'insertion du dispositif de mesure dans le matériau, ou après l'étape (b) de balayage en un gaz de la chambre gazeuse au moyen du système de balayage en un gaz à pression constante si celle-ci est présente. La pression désirée peut être toute pression adaptée à la mesure à réaliser. L'augmentation ou la diminution de pression de l'étape (c) peut être réalisée progressivement jusqu'à fracturer le matériau, notamment lorsque la paramètre à mesurer est le point de fracture. Par exemple, l'augmentation ou la diminution peut être suffisante pour atteindre une pression comprise entre -1 kPa et +200 kPa. Dans le cas d'une augmentation de la pression, le moyen d'entrée du gaz de balayage peut être ouvert afin de permettre l'entrée de gaz dans la chambre gazeuse, tandis que le moyen de sortie est fermé pour permettre l'augmentation de la pression dans la chambre gazeuse. Dans le cas d'une diminution de la pression, le moyen d'entrée du gaz de balayage peut être fermé et le moyen de sortie ouvert afin de permettre la sortie de gaz de la chambre gazeuse et ainsi permettre la diminution de la pression dans la chambre gazeuse. Bien entendu, il est possible d'obtenir des variations de pression par d'autres méthodes, par exemple en jouant sur la différence entre les débits d'entrée et de sortie du gaz.

L'étape (d), réalisée après l'étape de variation de la pression de la chambre gazeuse, peut être réalisée pendant une durée adaptée à la mesure souhaitée. L'homme du métier saura adapter cette durée au regard de ses connaissances générales. Par exemple, la durée de mesure peut être comprise entre quelques minutes et quelques jours, par exemple entre 2 minutes et 3 jours, voire plus si nécessaire. La fréquence d'acquisition de la pression est fonction de la variation en pression, par exemple selon la vitesse de production de gaz par le matériau.

Avantageusement, au moins une étape parmi les étapes (b) et (c) peut être réalisée au moins 2 fois de manière à réaliser un suivi de l'évolution de la pression dans le temps.

De manière avantageuse, le procédé de l'invention peut comprendre en outre une étape de calibration de la mesure en fonction du matériau et du gaz.

La température moyenne à laquelle les mesures sont réalisées peut être comprise entre -20°C, dans le cas d'une conservation à froid, et 200°C, dans le cas d'un procédé de cuisson. Par exemple, la température moyenne peut être la température ambiante, c'est-à-dire environ $20\pm1.5$ °C.

Le nombre de mesures réalisées est fonction des besoins de l'utilisateur. L'homme du métier saura adapter ce nombre, qui peut être par exemple compris entre 1 et 10, voire plus si besoin. Par exemple, dans le cas d'un fromage, le nombre de mesure peut être fonction du nombre de jours d'affinage.

Un autre objet de l'invention se rapporte à une utilisation d'un dispositif de mesure tel que défini précédemment, pour la mesure, notamment dans un matériau tel que défini précédemment, d'au moins une propriété physico-chimique choisies parmi des propriétés de transport de matière vis-à-vis de gaz et des propriétés mécaniques.

Les propriétés de transport de matière sont choisies parmi le coefficient de diffusion du gaz, la constante d'équilibre gaz gazeux/gaz dissout, la concentration en gaz dissous et/ou la vitesse de production.

Les propriétés mécaniques sont choisies parmi l'élasticité, la viscosité, la visco-élasticité et le point de fracture.

Un autre objet de l'invention se rapporte à l'utilisation d'un dispositif de mesure tel que défini précédemment, dans la préparation ou le suivi des caractéristiques de matériaux dans lesquels un gaz est susceptible de se solubiliser et de diffuser. Par exemple, ces matériaux et ces gaz sont tels que décrits précédemment.

Notamment, le dispositif de l'invention peut être uilisé comme un alvéographe pour caractériser un matériau tel que défini précédemment, par exemple de la pâte à pain.

D'autres avantages pourront encore apparaître à l'homme du métier à la lecture des exemples ci-dessous, illustrés par les figures annexées, donnés à titre illustratif.

## BRÈVE DESCRIPTION DES FIGURES

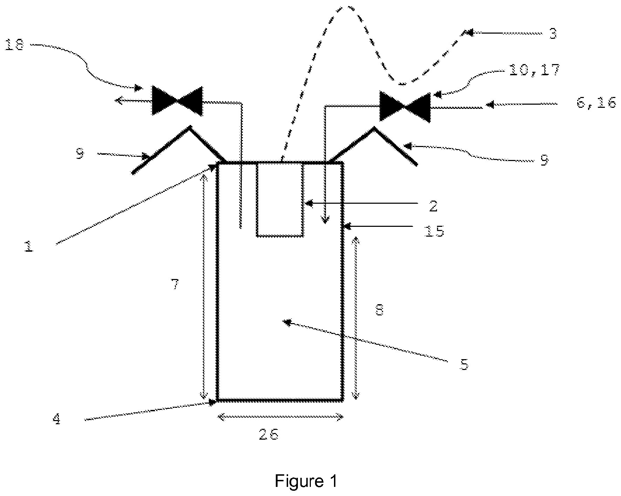

La figure 1 représente la coupe longitudinale d'un schéma de principe d'un dispositif de mesure selon l'invention, également appelé « sonde » ci-après.

Un capteur de pression (2) (Kulite Semiconductor Produtcs Inc, modèle XCQ-093-1.7.BARA) est inséré dans un tube (15) creux, cylindrique, de diamètre (26) et de hauteur (8), préférentiellement métallique ou en verre afin de ne pas absorber le $CO_2$. Le capteur de pression (2) est relié à un appareil (3) de traitement et d'enregistrement du signal.

L'étanchéité est garantie au niveau de l'extrémité supérieure (1) du dispositif par une soudure réalisée entre le capteur de pression (2) et le tube métallique. L'extrémité inférieure (4) du dispositif est ouverte puisque le tube (15) est creux, et est au contact de la matrice alimentaire à analyser.

Un système (6) de balayage en $CO_2$, nécessaire pour s'assurer que le milieu est uniquement composé de ce gaz, est aussi présent. Il consiste en un moyen (10) d'entrée en gaz par l'extrémité supérieure (1) du dispositif, reliée à une bouteille (16) de gaz et d'un moyen de sortie pour chasser le gaz.

Une vanne (17) permettant l'entrée dudit gaz dans le dispositif, et une vanne (18) permettant la sortie dudit gaz hors du dispositif, permettent de gérer l'entrée/la sortie en gaz. Le moyen (10) d'entrée en gaz permet également de régler la pression initiale à imposer pour réaliser les mesures.

Enfin, le dispositif est muni d'un moyen (9) de maintien en position à une matrice alimentaire pour assurer son maintien en position lors des mesures, qui consiste dans ce mode de réalisation en des pinces.

Dans ce mode de réalisation, le dispositif a les dimensions suivantes : Hauteur (7) =40 mm, hauteur (8) =7 mm, Diamètre (26) =3 mm.

Dans un autre mode de réalisation, non représenté sur les figures, pour faire les mesures, le balayage en $CO_2$ a été réalisé en amenant du gaz par l'extrémité inférieure (4) de la sonde (seule ouverture disponible) pendant au moins 1 minute avant de la planter dans le fromage.

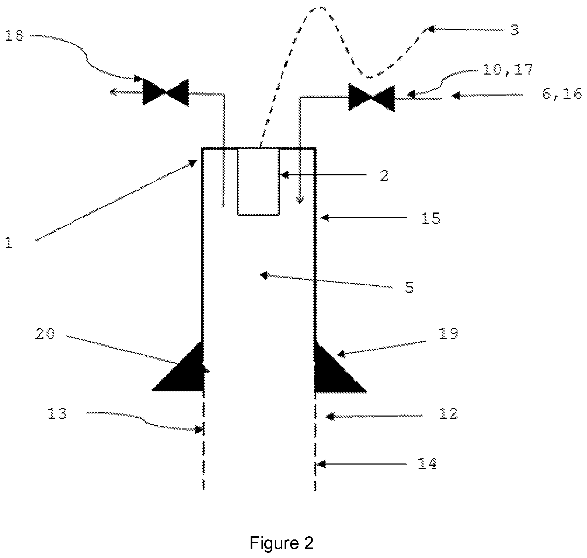

La figure 2 représente la coupe longitudinale d'un schéma de principe du dispositif de mesure du type de celui représenté sur la Figure 1, utilisé avec une extension (12). L'extension (12) représentée consiste en un tube métallique creux, ouvert à son sommet (20). Ce tube est éventuellement percé sur ses côtés (13) pour laisser passer le gaz et/ou percé en son extrémité inférieure (14) pour laisser passer le gaz. L'extension (12) est aussi munie d'un système (19) qui assure l'étanchéité avec la matrice à analyser (joint, système de vissage...). L'extension (12) pourra être vissée, accrochée au reste de la sonde.

La figure 3 représente la coupe longitudinale d'un schéma de principe du dispositif de mesure utilisé avec une extension (12) du type de celui représenté dans la Figure 2, inséré dans un trou (25) cylindrique réalisé dans la matrice (26) à analyser. Elle est insérée de manière à ce que le système (19) d'étanchéité de l'extension (12) est mis en position pour empêcher les fuites de gaz. Une chambre gazeuse (5) libre est présente entre la surface de la matrice à analyser et l'extension (12) de la sonde. Un espace gazeux est ménagé dans le trou (25) entre l'extension et la matrice.

Les figures 4 à 7 montrent les résultats des 4 mesures (respectivement figure 4, figure 5, figure 6 et figure 7) de pression (en kPa) effectuées en fonction du temps (heures). Sur chacune de ces figures, des droites ont été tracées en pointillés (respectivement « Mesure 1a » et « Mesure 1b » sur la figure 4, « Mesure 2 » sur la figure 5, « Mesure 3a » et « Mesure 3b » sur la figure 6 et « Mesure 4a » et « Mesure 4b » sur la figure 7) pour symboliser les durées utilisées pour calculer les vitesses de production en $CO_2$.

La figure 8 représente la vitesse de production en $CO_2$ ($r_{CO_2}$, en mol.m$^{-3}$.s$^{-1}$) par le fromage en fonction de la durée d'affinage (en jours), pour les données expérimentales obtenues (étoiles), les données mentionnées dans Huc et al. ([1]) (courbe en pointillés) et les données mentionnées dans Acerbi et al. ([2]) (courbe avec des tirets).

La figure 9 représente la mesure de pression en $CO_2$ (kPa) en fonction du temps (secondes) avec une sonde hermétique (pointillés) ou non hermétique (carrés). On voit clairement qu'avec une sonde non hermétique, la diminution de pression est beaucoup plus rapide et que la pression revient à sa valeur initiale après quelques minutes.

La figure 10 représente la mesure de la pression en $CO_2$ (kPa) en fonction du temps (secondes) avec le dispositif de l'invention avec un morceau de fromage (pointillés) et sans fromage avec un bouchon en plastique pour fermer l'extrémité inférieure du dispositif (carrés). On voit qu'avec un dispositif en matière plastique, la pression en $CO_2$ diminue rapidement due au transfert du $CO_2$ dans le plastique.

La figure 11 représente la mesure de la pression en $CO_2$ (kPa) en fonction du temps (secondes), effectuée 3 fois, après une surpression de la chambre gazeuse au contact du matériau. Les mesures ont été réalisées sur une durée d'environ 10 min (600s) avec une mesure de pression toutes les 10 secondes. Avant de faire les mesures, la chambre gazeuse avait été balayé en $CO_2$ pendant environ 1 minute.

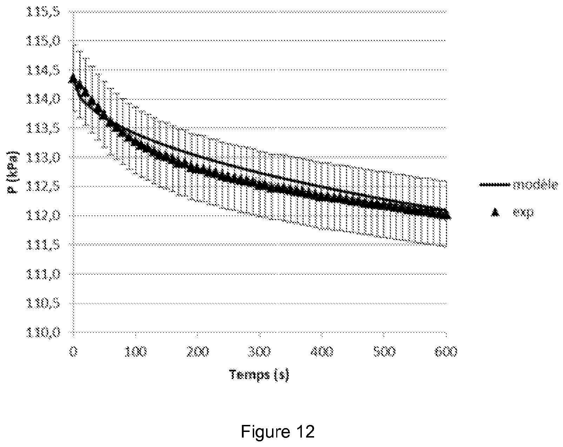

La figure 12 représente la mesure de la pression en $CO_2$ (kPa) en fonction du temps (secondes), pour les données expérimentales (Mesure 3, courbe avec des losanges) en comparaison avec le modèle ajusté avec l'équation 2 ($D_{CO_2}$= 2,6 x 10$^{-10}$ m$^2$.s$^{-1}$). Des barres d'erreur de 0,5 % ont été utilisées pour les valeurs expérimentales.

La figure 13 représente la coupe longitudinale d'un schéma de principe du dispositif de mesure du type de celui représenté sur la Figure 1, avec le système (6) de balayage en fonctionnement c'est-à-dire avec la vanne (17) d'entrée du gaz et la vanne (18) de sortie du gaz ouvertes. Le dispositif est planté dans la matrice (26) à analyser à une hauteur (22) >3 mm.

La figure 14 représente une mesure de la pression en $N_2$ (kPa) en fonction du temps (secondes), pour déterminer la viscosité d'un fromage.

La figure 15 représente la visosité (Pa.s) calculée avec l'équation 8 toutes les minutes lors du suivi de la pression. On voit clairement qu'après quelques minutes, la viscosité calculée se stabilise car le transfert de matière devient négligeable.

La figure 16 représente un schéma d'une sonde de mesure pour déterminer la constante de Henry (capteur (27) de température - capteur (2) de pression).

La figure 17 représente le principe de la variation de pression pour la mesure de la constante de Henry.

La figure 18 représente un schéma de la sonde de mesure utilisée pour déterminer la viscosité (capteur (27) de température - capteur (2) de pression).

La figure 19 représente la viscosité ($\mu$ en Pa.s) du bitume Azalt 70/100 déterminée avec la sonde (barres verticales, 9 mesures) et valeur de la littérature (trait horizontal en pointillés).

La figure 20 représente : a) Allure typique de la pression (kPa) au cours du temps (s) pour caractériser une pâte à pain obtenue avec un alvéographe - b) Allure typique de la pression au cours du temps pour caractériser une pâte à pain obtenue avec la sonde.

La figure 21 représente un schéma de la sonde de mesure utilisée pour déterminer les caractéristiques de la pâte à pain (capteur (27) de température - capteur (2) de pression).

La figure 22 représente la variation de pression (kPa) au cours du temps (s) pour caractériser la pâte à pain avec la sonde.

La figure 23 représente un schéma de la sonde de mesure utilisée pour déterminer de coefficient de diffusion du $CO_2$ dans l'eau (capteur (27) de température - capteur (2) de pression).

La figure 24 représente la pression (kPa) expérimentale et pression (kPa) déterminée avec l'équation (5) de l'exemple 11 concernant la diffusion du $CO_2$ dans l'eau, en fonction du temps (s).

**EXEMPLES**

**Exemple 1 : Détermination de la vitesse de production en $CO_2$ d'une matrice déformable**

La vitesse de production en $CO_2$ d'un fromage a été déterminée pendant les 11 premiers jours d'affinage à l'aide de mesure de pression uniquement. Le dispositif de mesure utilisé est cylindrique de hauteur (8) =7,0 mm et de diamètre (26) =2,7 mm, du type de celui décrit sur la Figure 1.

Equations utiles à la détermination de la vitesse de production en $CO_2$ d'une matrice déformable

L'équation (1) est le bilan de matière en $CO_2$ dans un fromage à pâte semi-dure de type Emmental. Le transport de matière par diffusion et la production de $CO_2$ (par fermentation propionique) sont pris en compte.

$$\frac{\partial c}{\partial t} - D_{CO2} \times \nabla C = r_{CO2} \tag{1}$$

Avec C la concentration en $CO_2$ ($mol.m^{-3}$), $D_{CO2}$ le coefficient de diffusion du $CO_2$ dans le fromage ($m^2.s^{-1}$) et $r_{CO2}$ la vitesse de production en $CO_2$ ($mol.m^{-3}.s^{-1}$) L'équation 2 représente l'équilibre thermodynamique du CO2 à l'interface entre une phase gaz et une phase liquide (eau+gras du fromage). L'équilibre est basé sur l'équation d'Henry.

$$C = k_H^{ch} \times P_{CO2} \qquad (2)$$

Avec $k_H^{ch}$ la constant de Henry (mol.m$^{-3}$.Pa$^{-1}$) et $P_{CO2}$ la pression en $CO_2$ dans la phase gaz (Pa).
Pour déterminer la vitesse de production de $CO_2$, on considère la diffusion très lente ($D_{CO2}$ très petit) devant la production de $CO_2$. Dans ce cas, l'équation 1 devient :

$$\frac{\partial c}{\partial t} = r_{CO2} \qquad (3)$$

En couplant l'équation 3 avec l'équation 2 (loi de Henry) et en intégrant selon le temps, on obtient l'équation 4 qui décrit l'augmentation de pression en fonction du temps de manière linéaire. La pente de l'augmentation de pression due à la production de $CO_2$ en fonction du temps permet d'obtenir la vitesse de production de $CO_2$ en connaissant la constante de Henry uniquement.

$$P = P_0 + \frac{r_{CO2}}{k_h} \times t \qquad (4)$$

Principe des mesures

Le principe des mesures est de planter le dispositif dans le fromage et de suivre l'évolution de la pression au cours du temps. En plantant le dispositif dans le fromage, le volume de la phase gaz diminue, ce qui augmente la pression à une valeur P0 supérieure à la pression atmosphérique. Une baisse de pression est d'abord observée due au transfert du $CO_2$ de la phase gaz vers le fromage jusqu'à atteindre un équilibre (selon l'équation 2). Une fois l'équilibre atteint, la pression va ensuite augmenter due à la production de $CO_2$ par le fromage et son transfert vers la phase gaz. C'est à partir de cette augmentation de pression que la vitesse de production en $CO_2$ est déterminée selon l'équation 4.

Conditions opératoires

Un fromage à pâte semi-dure de type Emmental est utilisé pour déterminer la vitesse de production en $CO_2$. Pour chaque mesure, un trou (25) est réalisé dans le fromage afin de faire une mesure à cœur (à 2 cm de profondeur). Pour assurer une atmosphère en $CO_2$, la sonde est balayée avec du $CO_2$ pendant 1 minute avant de la planter dans le fromage.
Les mesures durent entre 1 et 2 jours (10 secondes ou 1 minute de fréquence d'acquisition de la pression) selon l'augmentation en pression (c'est-à-dire selon la vitesse de production). La température moyenne dans la pièce était de 20 $\pm$1.5 °C. Quatre mesures ont été effectuées, ce qui a couvert les 11 premiers jours d'affinage.

Résultats expérimentaux de mesures de pression

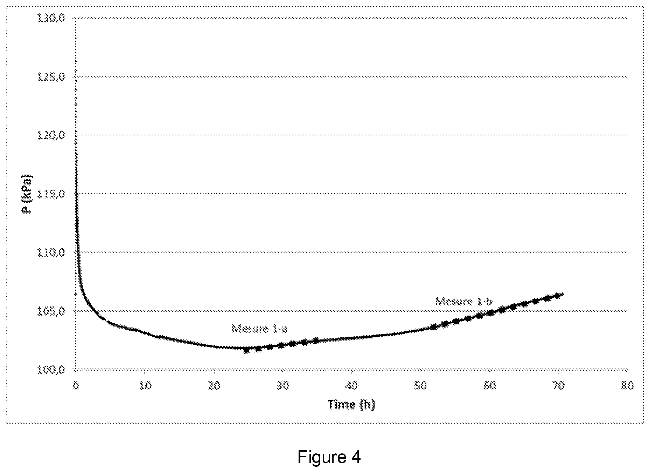

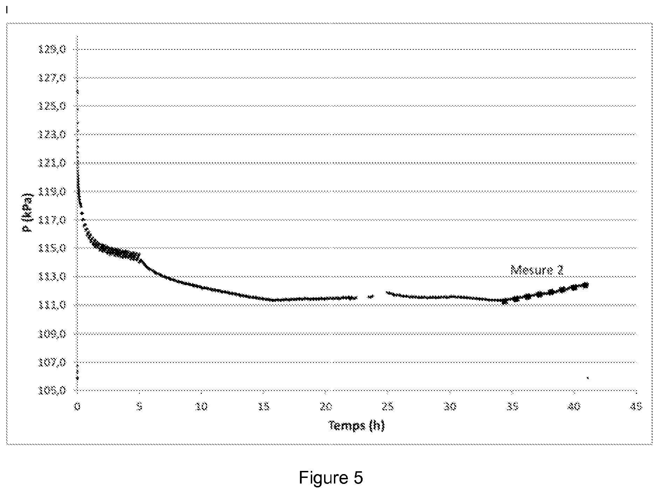

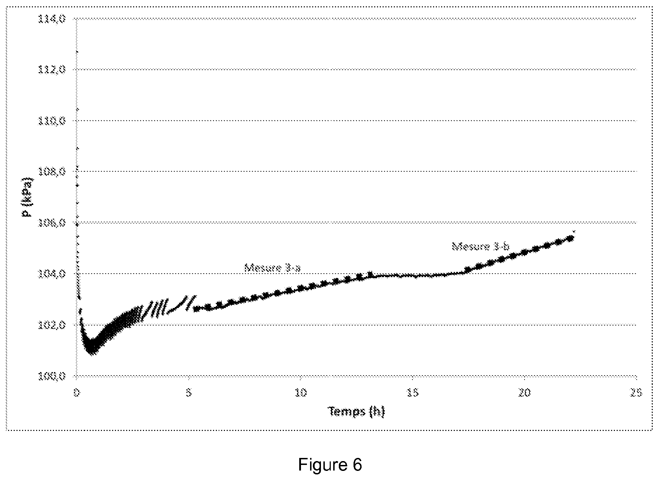

Les figures 4 à 7 montre les résultats des mesures de pression effectuées. Chaque courbe a la même allure : comme expliqué précédemment, la pression diminue dans un premier temps après la mise en surpression due au transfert de matière du $CO_2$ de la phase gaz vers le fromage ; un pallier est ensuite atteint, puis la pression ré-augmente suite à la production et au transfert du $CO_2$ vers la phase gaz cette fois.
Sur les figures 4 à 7, des droites ont aussi été tracées lors des périodes où la pression ré-augmente. Elle symbolise les données avec lesquelles les vitesses de production en $CO_2$ ont été déterminées (la pente de ces droites permet de calculer la vitesse de production avec l'équation 4).

Détermination des vitesses de production en $CO_2$

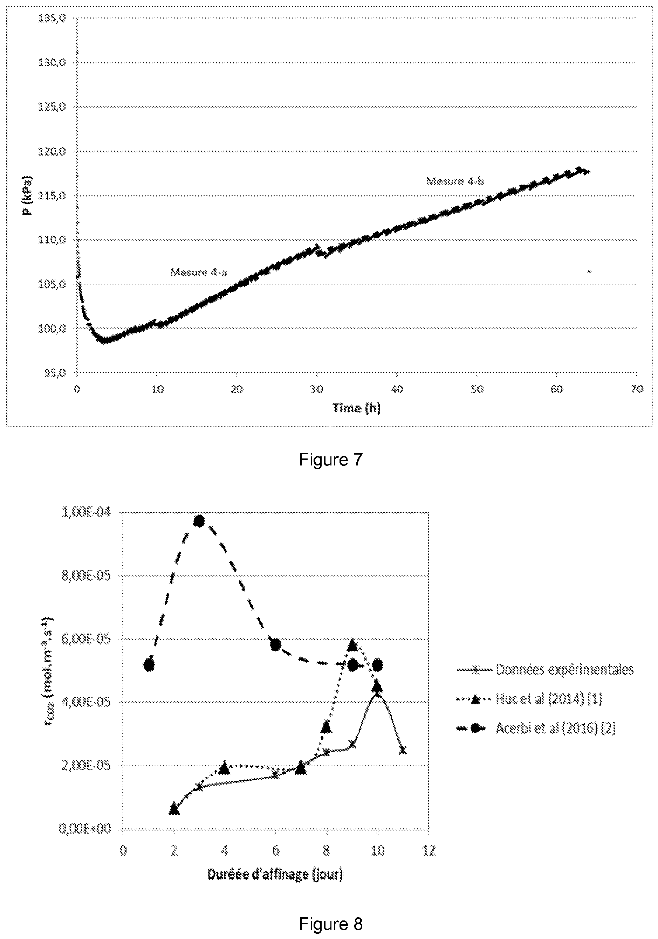

A partir des données expérimentales présentées dans le paragraphe précédent, les vitesses de production en $CO_2$ ont été calculées avec l'équation 4 en prenant comme valeur de la constante de Henry 3,5 x 10$^{-4}$ mol.m$^{-3}$.Pa$^{-1}$ (Chaix et al [7] ; Chaix E. [6]). Les résultats sont regroupés dans le Tableau 1 et sont aussi présentés figure 8.
La vitesse de production est la plus faible au début de l'affinage (jour 2). Elle augmente ensuite progressivement jusqu'au jour 10 ou elle atteint un maximum puis rediminue au jour 11.
La Tableau 1 représente les vitesses expérimentales d'augmentation en pression et vitesses de production en $CO_2$ correspondantes déterminées avec l'équation 4 (constante de Henry égale à 3,5 x 10$^{-4}$ mol.m$^{-3}$.Pa$^{-1}$)

[Table 1]

| Référence de la mesure | Jour d'affinage | Pente de la droite qui représente l'augmentation de pression (Pa.s$^{-1}$) | **Vitesse de** production en $CO_2$ calculée avec l'équation 4 (mol.m$^{-3}$.s$^{-1}$) |
|---|---|---|---|
| Mesure 1-a | 2 | $1{,}63 \times 10^{-2}$ | $5{,}70 \times 10^{-6}$ |
| Mesure 1-b | 3 | $3{,}74 \times 10^{-2}$ | $1{,}31 \times 10^{-5}$ |
| Mesure 2 | 6 | $4{,}84 \times 10^{-2}$ | $1{,}70 \times 10^{-5}$ |
| Mesure 3-a | 8 | $6{,}91 \times 10^{-2}$ | $2{,}42 \times 10^{-5}$ |
| Mesure 3-b | 9 | $7{,}62 \times 10^{-2}$ | $2{,}67 \times 10^{-5}$ |
| Mesure 4-a | 10 | $12{,}20 \times 10^{-2}$ | $4{,}26 \times 10^{-5}$ |
| Mesure 4-b | 11 | $7{,}09 \times 10^{-2}$ | $2{,}48 \times 10^{-5}$ |

Comparaison avec les données de la littérature.

La figure 8 présente la vitesse de production en $CO_2$ par le fromage en fonction de la durée d'affinage (données du Tableau 1) ainsi que quelques valeurs de la littérature pour comparaison.

D'une manière générale, les vitesses de production déterminée dans cette étude sont cohérentes avec les valeurs de la littérature (Huc et al. : « Influence of salt content on eye growth in semi-hard cheese studied using magnetic resonance imaging and $CO_2$ production measurements », International Dairy Journal (2014) ([1]) ; Acerbi et al. ([2])). Le maximum de production est obtenu après 10 jours d'affinage par Huc et al. ([1]) pour des fromages du même type que celui testé. Par contre, Acerbi et al. ([2]) ont déterminé un maximum de production en $CO_2$ après 3 jours d'affinage.

Conclusion

Les vitesses de production en $CO_2$ déterminées au moyen du dispositif de l'invention sont cohérentes avec les valeurs de la littérature (de l'ordre de $10^{-6}$ - $10^{-5}$ mol.m$^{-3}$.s$^{-1}$). La vitesse augmente les 10 premiers jours d'affinage (jusqu'à un maximum de $4{,}26 \times 10^{-5}$ mol.m$^{-3}$.s$^{-1}$) puis diminue ensuite.

**Exemple 2 : Détermination du coefficient de diffusion de $CO_2$ d'une matrice déformable**

Dans cet exemple, pour déterminer les propriétés du fromage, on utilise un dispositif du type de celui défini sur la figure 1, disposant d'un capteur de pression (2) d'une phase gazeuse uniquement avec du $CO_2$ dont le volume est petit devant celui du fromage (fromage semi-infini, longueur phase gaz e (correspondant à la hauteur de la phase gaz après avoir enfoncé la sonde) << profondeur d'insertion du dispositif dans le fromage L. Par ailleurs, on néglige la production de $CO_2$ par fermentation et on considère qu'il n'y a pas de compression du fromage due à la pression.

Ce dispositif est un tube cylindrique de hauteur (8) =7,0 mm et de diamètre (26) =2,7 mm constitué d'étain. L'étanchéité est assurée en soudant le capteur de pression (2) au tube en étain.

Equations utiles à la détermination du coefficient de diffusion de $CO_2$ d'une matrice déformable

Dans ces conditions et en supposant que le transfert se fait uniquement dans une direction, l'équation du transport de matière est (équation 1) :

$$\frac{\partial c}{\partial t} - D_{CO2} \times \frac{\partial^2 c}{\partial x^2} = 0 \qquad\qquad (1)$$

Pour résoudre cette équation, on suppose que la concentration initiale en $CO_2$ dans le fromage est homogène dans le fromage et égale à $C_0$. On impose une pression $P_0$ supérieure à la pression atmosphérique au temps t=0. Les conditions limites et initiales sont donc :

En x=0 et t=0 $\quad C = k_H^{ch} \times P_0$ (loi de Henry pour déterminer l'équilibre à l'interface gaz/liquide)

En x>0 et t=0 : $\quad C = C_0$

$$\text{En } x=L : \frac{\partial c}{\partial x} = 0$$

Cette équation a une solution analytique pour une plaque semi-infinie (Tveteraas O.: "A study of pressure decay in a closed CO2-water system", Master Thesis, 2011 ([3]), Ghaderi et al.: "Estimation of concentration-dependent diffusion coefficient in pressure-decay experiment of heavy oils and bitumen", Fluid phase equilibria, 2011 ([4])) :

$$P(t) = \frac{C_0}{k_h} + \left(P_0 - \frac{C_0}{k_h}\right) \times \exp\left[\frac{t}{D_{CO2}} \times \left(\frac{R \times T \times D_{CO2} \times k_h}{e}\right)^2\right] \times \text{erfc}\left[\sqrt{\frac{t}{D_{CO2}}} \times \left(\frac{R \times T \times D_{CO2} \times k_h}{e}\right)\right] \quad (2)$$

A partir de l'équation 2, il est donc possible de décrire l'évolution de la pression en $CO_2$ en fonction de la concentration initiale en $CO_2$, de la constante de Henry et du coefficient de diffusion du $CO_2$. Par ajustement avec des données expérimentales de mesures de pression, il serait possible de déterminer aussi ces paramètres.

Remarque : La fonction erfc est comprise entre 2 et 0 avec des valeurs qui tendent vers 2 lorsque l'argument tend vers -∞ et elle tend vers 0 lorsque l' argument tend vers +∞.

A partir de l'équation 2, deux comportements caractéristiques peuvent être dégagés :

Le comportement sur un temps très long :

$$\lim_{t \to +\infty} P(t) = \frac{C_0}{k_h} \qquad (3)$$

Le comportement sur un temps court :

$$\lim_{t \to 0} P(t) = \frac{C_0}{k_h} + \left(P_0 - \frac{C_0}{k_h}\right) \times \left[1 - \frac{2}{\sqrt{\pi}} \times \frac{\sqrt{t}}{\sqrt{D_{CO2}}} \times \frac{R \times T \times D_{CO2} \times k_h}{e}\right] \qquad (4)$$

L'équation 4 vient du produit des limites des fonctions exp et erfc

$$\left(\lim_{x \to 0} \exp(x) = 1 \text{ et } \lim_{x \to 0} \text{erfc}(x) = 1 - \frac{2}{\sqrt{\pi}} \times x\right).$$

L'équation 3 donne l'information sur la sursaturation du fromage lorsque la pression s'est stabilisée sur un temps très long. Néanmoins, le comportement sur un temps très long est difficile à utiliser en pratique car la production en $CO_2$ n'est plus négligeable (selon la durée d'affinage et les caractéristiques du fromage).

L'équation 4 décrit le comportement sur un temps de mesure court. En réalité cette équation peut être remise sous la forme d'une droite (équation 5).

$$\lim_{t \to 0} P(t) = \frac{C_0}{k_h} + \left(P_0 - \frac{C_0}{k_h}\right) \times \left[1 - \frac{2}{\sqrt{\pi}} \times \frac{\sqrt{t}}{\sqrt{D_{CO2}}} \times \frac{R \times T \times D_{CO2} \times k_h}{e}\right] = P_0 - \left(P_0 - \frac{C_0}{k_h}\right) \times \frac{2}{\sqrt{\pi}} \times \frac{\sqrt{t}}{\sqrt{D_{CO2}}} \times \frac{R \times T \times D_{CO2} \times k_h}{e} \qquad (5)$$

$$\lim_{t \to 0} P(t) = b - a \times \sqrt{t}$$

Avec $b = P_0$

$$a = -\left(P_0 - \frac{C_0}{k_h}\right) \times \frac{2}{\sqrt{\pi}} \times \frac{R \times T \times \sqrt{D_{CO2}} \times k_h}{e} \qquad (6)$$

L'équation 6 donne une seconde relation entre $C_0$, $k_H$ et $D_{CO2}$.

Mesure de l'étanchéité

Plusieurs soudures ont été réalisées entre le capteur de pression (2) et l'extrémité supérieure (1) du dispositif. A chaque fois, 2 tests d'étanchéités sont réalisées : (i) l'allure de la courbe de diminution de pression en $CO_2$ et (ii) en plongeant la sonde dans de l'eau et en injectant de l'air à l'aide d'une seringue.

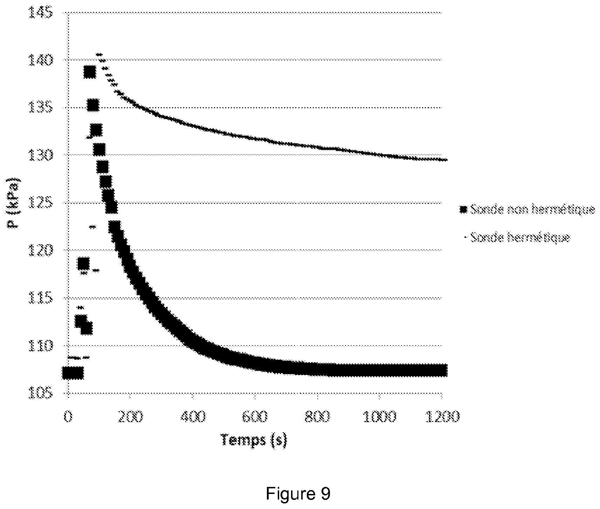

La figure 9 compare l'allure des courbes de diminution de pression en $CO_2$ avec une sonde hermétique ou non. On voit clairement qu'avec une sonde non hermétique, la diminution de pression est beaucoup plus rapide et que la pression revient à sa valeur initiale après quelques minutes.

Mise en évidence des matériaux pour construire la sonde

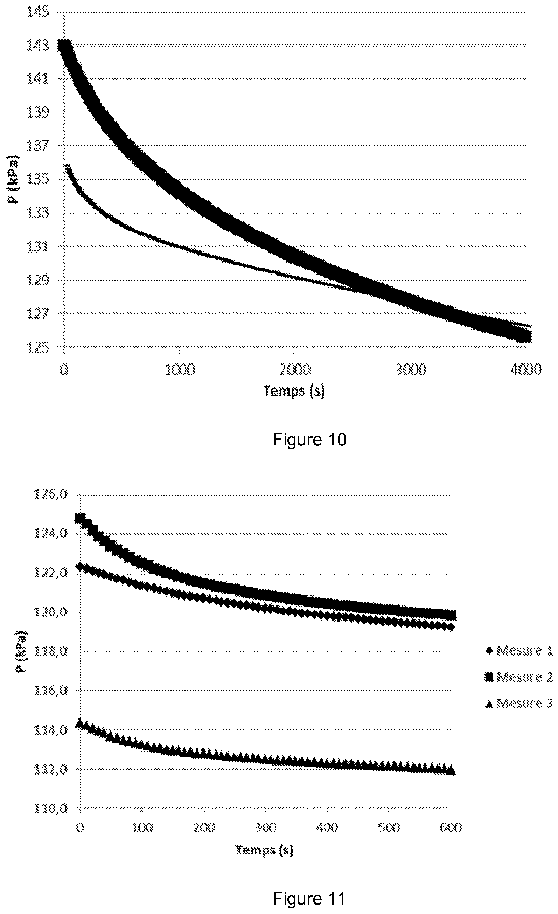

Des mesures de pression en $CO_2$ ont été réalisées avec le dispositif de l'invention, et avec un morceau de fromage et sans fromage mais avec un bouchon en matière plastique (non préalablement saturé en $CO_2$). La figure 10 compare les résultats obtenus dans les deux cas (avec le dispositif de l'invention avec un morceau de fromage en pointillés, et sans fromage avec un bonchon en plastique pour fermer l'extrémité inférieure du dispositif avec des carrés). On voit qu'avec un dispositif en matière plastique, la pression en $CO_2$ diminue rapidement due au transfert du $CO_2$ dans le plastique ce qui illustre qu'il est impératif d'utiliser des matières qui n'absorbent pas le gaz d'intérêt (le CO2 ici).

Principe des mesures de pression

Le principe est de planter le dispositif tel que représenté dans la figure 1 dans une matrice alimentaire. En plantant le tube dans le fromage, le volume de la phase gaz diminue, ce qui augmente la pression à une valeur $P_0$ supérieure à la pression atmosphérique. Une baisse de pression est ensuite observée en accord avec l'équation 2.

Pour assurer une atmosphère en $CO_2$, le dispositif est balayée avec du $CO_2$ pendant 1 minute avant de la planter dans le fromage.

Des mesures de pression en $CO_2$ ont été réalisées avec la sonde avec un fromage à pate semi-dure de type Emmental après 2 mois d'affinage. Compte tenu de la date de réalisation du fromage, on considère qu'il n'y a plus du tout de production de $CO_2$. La température moyenne dans la pièce était de 19,7 °C.

La sonde est plantée à cœur dans le fromage (à environ 2 cm de profondeur) et une potence permettait de caler la sonde. Les mesures ont été réalisés sur une durée d'environ 10 min (600 s) avec une mesure de pression toutes les 10 secondes. Une telle durée d'expérience est raisonnable avec un fromage fermentescible sans que la production de $CO_2$ ne modifie significativement les mesures de pression.

Résultats et discussion

Résultats expérimentaux de mesures de pression

La figure 11 montre les résultats expérimentaux de mesure de pression obtenus. Dans tous les cas, la pression diminue au cours du temps conformément à l'équation 2, ce qui confirme que du $CO_2$ transfère de la phase gaz vers le fromage. L'allure des courbes est la même pour toutes les mesures, quel que soit l'endroit de la mesure et la pression initiale imposée.

L'objectif est de déterminer le coefficient de diffusion du $CO_2$ dans le fromage et la concentration initiale en $CO_2$. On considère que la constante de Henry $k_H$ est connue et égale à $3,5 \times 10^{-4}$ mol.m$^{-3}$.s$^{-1}$ (Chaix et al [7]). Les 2 paramètres sont obtenus par ajustement des équations 2 et 6 avec les données expérimentales. Pour utiliser l'équation 2, il faut connaître la hauteur de la phase gaz e. Ce paramètre est obtenu avec la hauteur initiale h (7,0 mm) et la pression initiale imposée $P_0$ selon l'équation 7.

$$e = \frac{h \times P_{atm}}{P_0} \qquad\qquad (7)$$

La Figure 9 compare le modèle avec les données expérimentales (Mesure 3). Des barres d'erreur de 0,5 % ont été utilisées pour les valeurs expérimentales. Le modèle est en bon accord avec les valeurs expérimentales avec un coefficient de diffusion du $CO_2$ de $2,6 \times 10^{-9}$ m$^2$.s$^{-1}$ et une concentration en $CO_2$ dissout de 26 mol.m$^{-3}$. Compte-tenu de la valeur de la constante de Henry, une telle concentration indique que le fromage est sous-saturé en $CO_2$, ce qui est cohérent après un affinage de plus de 2 mois et avec un fromage en contact avec l'air ambient pendant certaines périodes.

Le Tableau 2 récapitule les résultats obtenus et les compare avec les données de la littérature. Les coefficients de diffusion obtenus sont compris entre $2,6 \times 10^{-10}$ et $5,7 \times 10^{-10}$ m$^2$.s$^{-1}$. Ces valeurs sont cohérentes avec celles de la littérature (Acerbi et al [5]). Le Tableau 2 représente les valeurs des coefficients de diffusion ajustés ($k_H = 3,5 \times 10^{-4}$ mol.m$^{-3}$.Pa$^{-1}$) et valeur de la littérature.

[Table 2]

| Référence | $C_0$ (mol.m$^{-3}$) | tableau |
|---|---|---|
| Mesure 1 | 26 | **3,0** x 10$^{-10}$ |
| Mesure 2 | 25 | 5,7 x 10$^{-10}$ |
| Mesure 3 | 26 | **2,6** x 10$^{-10}$ |
| Moyenne | 26 | **3,8** x 10$^{-10}$ |
| Acerbi et al [5] | - | **6,8** x 10$^{-11}$ |

Conclusion

A partir de mesures de variation de pression en $CO_2$ dans une phase gaz au contact d'une matrice alimentaire (fromage) il a été possible de déterminer certaines caractéristiques de la matrice alimentaire (concentration initiale en $CO_2$ et coefficient de diffusion du $CO_2$) par ajustement d'un modèle avec les données expérimentales.

Les mesures sont réalisées pendant 10 minutes. Plusieurs précautions expérimentales ont été mises en avant :

Il est nécessaire de ne pas laisser le fromage au contact de l'air libre pour éviter la désolubilisation du $CO_2$ qui altère la qualité des paramètres ajustés.

Il ne faut pas utiliser de matière plastique pour le design de la sonde car ces dernières absorbent le $CO_2$.

Une sonde, entièrement métallique, a été fabriquée au laboratoire. En supposant la constante de Henry connue (3,5 x 10$^{-4}$ mol.m$^{-3}$.Pa$^{-1}$), on a déterminé que la valeur moyenne du coefficient de diffusion est de 3,8 x 10$^{-10}$ m$^2$.s$^{-1}$. Cette valeur est en bon accord avec les résultats de la littérature.

**Exemple 3** : **Détermination des propriétés de transport de matière d'une matrice déformable au moyen du dispositif de l'invention**

Dans cet exemple, pour déterminer les propriétés du fromage, on utilise un dispositif du type de celui défini sur la figure 1.

Qu'elle que soit la propriété de transport à déterminer, le principe de la mesure reste le même que pour les exemples précédents, et il est décrit ci-dessous :

1) Le dispositif, à l'air libre, est balayée par le gaz d'intérêt, ici du $CO_2$. La vanne (18) permettant la sortie dudit gaz hors du dispositif est ouverte, la vanne (17) permettant l'entrée dudit gaz dans le dispositif est ouverte aussi.

2) Un trou (25) est fait dans la matrice à analyser, comme dans le présent exemple, qu'il s'agisse d'une matrice alimentaire ou d'un autre matériau, pour pouvoir au besoin faire la mesure à cœur. Ce dernier peut par exemple être réalisé avec un foret.

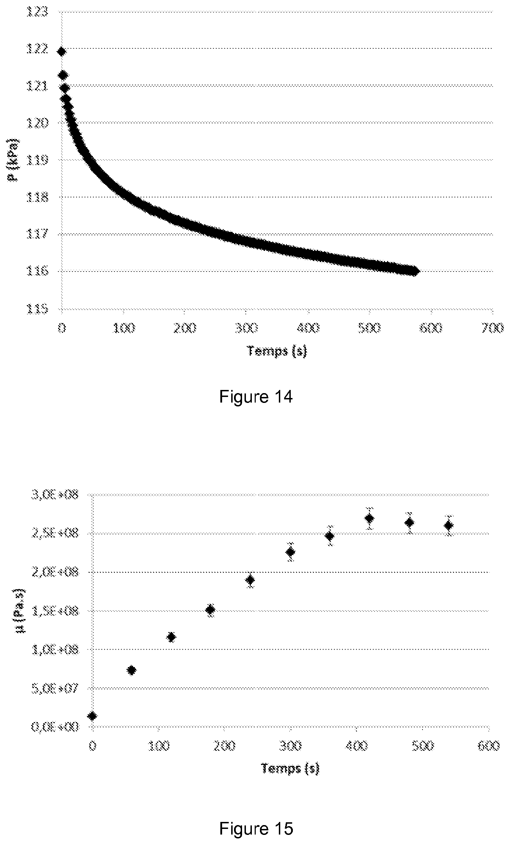

3) Le dispositif, avec le système (6) de balayage en fonctionnement c'est-à-dire avec la vanne (17) d'entrée du gaz et la vanne de sortie du gaz (18) ouvertes, est plantée dans la matrice à analyser. Elle est plantée à une hauteur (22), qui est au minimum de 1 mm dans la matrice (pour respecter certaines hypothèses de calcul pour le traitement des données) et préférentiellement à une hauteur (22) >3 mm, comme représenté sur la figure 14.

Il est nécessaire que la face ouverte de la sonde soit celle par laquelle la sonde est plantée dans la matrice.

La sonde est insérée rapidement dans la matrice.

4) Le système (6) de balayage est maintenu pendant quelques dizaines de secondes avec la sonde plantée dans le fromage pour s'assurer que la matrice soit uniquement en contact avec le gaz d'intérêt.

Ce temps doit être proche du temps qui sépare la réalisation du trou (25) et la réalisation de la sonde.

5) Une fois le balayage fini, la vanne (18) permettant la sortie dudit gaz hors du dispositif est fermée.

6) La vanne (17) d'entrée du gaz alimente alors la chambre gazeuse (5) au contact de la matrice jusqu'à atteindre rapidement la pression désirée (impulsion en pression). La pression à atteindre est entre -1 kPa et +200 kPa, préférentiellement +10-15 kPa.

7) Une fois la pression désirée atteinte, la vanne (17) est fermée. La vanne (18) reste fermée. La mesure de pression commence alors avec les vannes (17) et (18) fermées pendant toutes la durée de l'analyse.

**Exemple 4** : **Détermination expérimentale de la viscosité d'un fromage**

Dans cet exemple, pour déterminer les propriétés mécaniques du fromage en particulier sa viscosité, on utilise un dispositif du type de celui défini sur la figure 2, disposant d'une extension formée d'un tube cylindrique percé sur les côtés et en son extrémité inférieure.

Equations utiles à la détermination de la viscosité

La viscosité et la pression sont reliées à la déformation du rayon du cylindre (ce dernier est supposé infini) selon l'équation (1).

$$P_{cylindre} - P_{atm} = 2 \times \mu \times \frac{dR}{R} \times \frac{1}{dt} \qquad (1)$$

**Avec** $P_{cylindre}$ la pression dans le cylindre rempli de gaz (Pa), $P_{atm}$ la pression atmosphérique (Pa), $\mu$ la viscosité du fromage (Pa.s), R le rayon du cylindre (m) et dt l'intervalle de temps de mesure (s).

Principe de la détermination de la viscosité

Expérimentalement, un trou cylindrique est creusé dans le fromage et l'évolution de la pression au cours du temps suite à une surpression est mesurée. En accord avec la loi des gaz parfaits et en négligeant le transfert de matière par rapport à la mécanique, une diminution de pression correspond à une augmentation de volume du cylindre que l'on peut rapporter à une augmentation du rayon du cylindre (c'est-à-dire que la variation de rayon d'un cercle est proportionnelle à la variation de surface à la puissance 1/2, équation (2)).

$$\frac{\Delta R}{R} = \frac{P_1^{1/2} - P_2^{1/2}}{P_1^{1/2}} \qquad (2)$$

Avec $P_1$ la pression dans le cylindre à l'instant $t_1$ (Pa) et $P_2$ la pression dans le cylindre à l'instant t2>t1 (Pa).
Les mesures sont réalisées avec un fromage à pate semi-dure de type Emmental. Le gaz utilisé pour faire ces mesures est de l'azote qui a une faible solubilité dans des matrices avec beaucoup d'eau (c'est le cas du fromage étudié).
L'extension de la sonde a une hauteur de 20 mm. Pour faire les mesures, un trou cylidrique de hauteur 60 mm est creusé avec un petit foret puis la sonde est enfoncée dans le trou jusqu'en buté au niveau du capuchon qui assurait l'étanchéité du système avec l'extérieur. Deux poids métalliques (500 g chacun) sont ensuite mis en place pour assurer le maintien en position de la sonde.
Avant la mesure, la chambre gazeuse est balayée par de l'azote pendant environ 1 min. Une supression comprise entre +15 et +35 kPa est ensuite imposée et la pression est mesurée pendant environ 10 minutes pour chaque mesure avec un pas de temps de 1 seconde.

Résultats et discussion

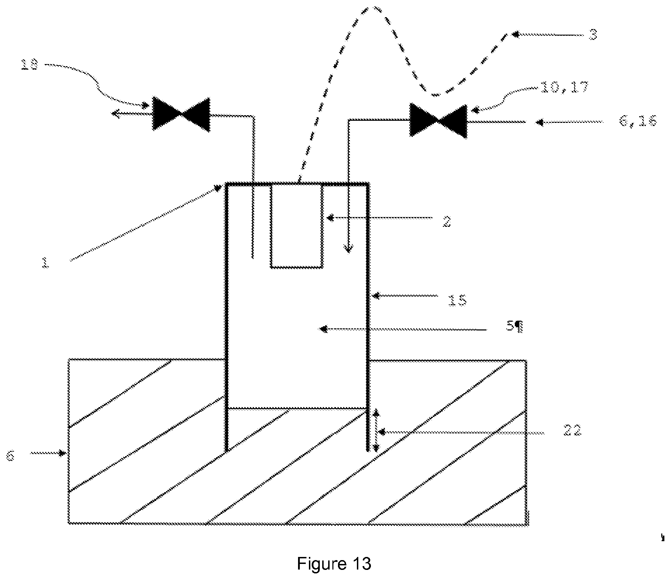

Pendant toute la durée de l'analyse, la pression a diminué due à l'augmentation de volume du trou cylindrique préalablement creusé et aussi dans les permiers instants de l'analyse au transfert du gaz de la phase gaz vers le fromage. La Figure 15 montre une allure typique de la pression au cours du temps.
La viscosité a été déterminée en considérant les valeurs de pression toutes les minutes car pendant cet intervalle de temps la pression évolue peu (Figure 15). Au début de l'analyse, la viscosité calculée est faible pour ce type de fromage et elle augmente au cours du temps (Figure 13). Ce comportement vient de la pression qui diminue à la fois par l'augmentation de volume selon la loi des gaz parfaits et aussi par le transfert du gaz dans le fromage. Après quelques minutes (environ 5 min), le transfert de matière devient très faible (la surface du fromage en contact avec la chambre gazeuse est saturée en azote) et négligeable par rapport au comportement mécanique et la viscosité calculée devient constante à une valeur d'environ 2,5 x $10^8$ Pa.s en bon accord avec la littérature (Grenier et al, 2016 [9]).

**Exemple 5** : **Détermination des propriétés mécaniques d'une matrice déformable au moyen du dispositif de l'invention**

Pour la détermination des propriétés mécaniques, le dispositif de mesure est utilisé avec une extension (12), comme représenté sur la figure 2.
Le principe de la mesure des propriétés mécaniques est décrit ci-dessous. Il diffère selon si la propriété à déterminer est la viscosité ou le point de fracture.

Détermination de la viscosité

1) La sonde avec son extension (12), à l'air libre, est balayée par du gaz. La vanne (17) est ouverte, la vanne (18) est ouverte aussi. Le gaz utilisé pour cette mesure est préférentiellement un gaz peu soluble dans la matrice à analyser pour mesurer des propriétés mécaniques et pas celles de transport. Par exemple, il est préférable d'utiliser de l'azote $N_2$ pour des matrices avec beaucoup d'eau (c'est le cas des fromages par exemple), afin de limiter son transfert dans la matrice à analyser.

2) Un trou (25), préférentiellement cylindrique, est réalisé dans la matrice à analyser. Il a préférentiellement une hauteur (23) minimale de 60 mm. Ce trou (25) peut par exemple être réalisé avec un foret.

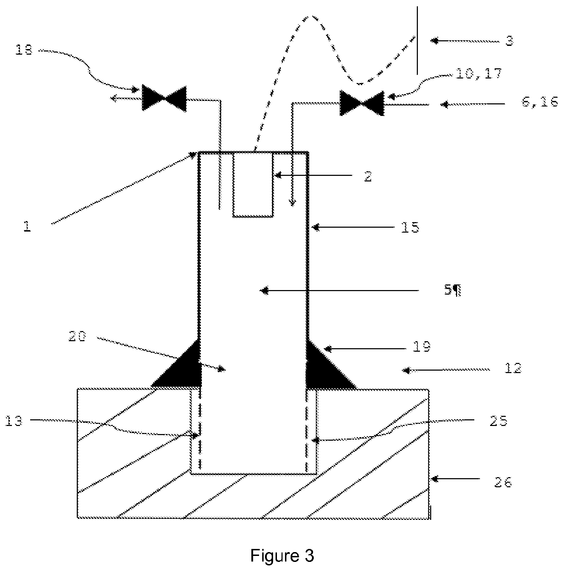

3) La sonde avec son extension (12), avec le système (6) de balayage en fonctionnement, c'est-à-dire avec les vannes (17) et (18) ouvertes, est insérée dans le trou (25) dans la matrice à analyser, comme représenté sur la figure 3.

Elle est insérée de manière à ce que le système (19) d'étanchéité de l'extension (12) soit mis en position pour empêcher les fuites de gaz.
Une chambre gazeuse (5) libre doit être présent entre la surface de la matrice à analyser et l'extension (12) de la sonde.

4) Le système (6) de balayage est maintenu pendant quelques dizaines de secondes avec la sonde plantée dans le fromage pour s'assurer que la matrice soit uniquement en contact avec le gaz d'intérêt.

5) Une fois le balayage fini, la vanne (18) de sortie est fermée.

6) La vanne (17) alimente la chambre gazeuse (5) au contact de la matrice jusqu'à atteindre rapidement la pression désirée (impulsion en pression). La pression à atteindre est comprise entre +1 kPa et +150 kPa.

7) Une fois la pression désirée atteinte, la vanne (17) d'entrée est fermée. La vanne (18) reste fermée. La mesure de pression commence alors avec les vannes (17) et (18) fermées pendant toutes la durée de l'analyse (quelques minutes).

Détermination du point de fracture

Le principe de fonctionnement de la sonde pour la détermination du point de fracture est le suivant :

1) La sonde avec son extension (12), à l'air libre, est balayée par du gaz. La vanne (17) est ouverte, la vanne (18) est ouverte aussi.
Le gaz utilisé pour cette mesure est préférentiellement un gaz peu soluble dans la matrice à analyser pour mesurer des propriétés mécaniques et pas celles de transport. Par exemple, il est préférable d'utiliser de l'azote N2 pour des matrices avec beaucoup d'eau, comme les fromages par exemple, afin de limiter son transfert dans la matrice à analyser.

2) Un trou (25), possiblement cylindrique, est réalisé dans la matrice à analyser. Ce trou (25) peut par exemple être réalisé avec un foret.

3) Le dispositif avec son extension (12), avec le système (6) de balayage en fonctionnement c'est-à-dire avec les vannes (17) et (18) ouvertes, est insérée dans le trou (25) dans la matrice à analyser.

Elle est insérée de manière à ce que le moyen (19) d'étanchéité de l'extension (12) soit mis en position pour empêcher les fuites de gaz.
Une chambre gazeuse (5) libre doit être présent entre la surface de la matrice à analyser et l'extension (12) de la sonde, comme montré sur la Figure 3.

4) Le système (6) de balayage est maintenu pendant quelques dizaines de secondes avec la sonde plantée dans le fromage pour s'assurer que la matrice soit uniquement en contact avec le gaz d'intérêt.

5) Une fois le balayage fini, la vanne (18) est fermée.

6) La vanne (17) alimente la chambre gazeuse (5) au contact de la matrice à analyser. La pression augmente progressivement jusqu'à « fracturer » la matrice, moment à partir duquel la pression revient à la pression atmosphérique.

**Exemple 6: détermination de la constante de Henry du $CO_2$ vis-à-vis d'un fromage**

1. Théorie

Pour déterminer la constante de Henry $k_H$ d'un gaz vis-à-vis d'une matrice à caractériser, on place un morceau de la matrice de volume connu $V_{matrice}$ dans une enceinte fermée de volume $V_{enceinte}$ contenant uniquement le gaz d'intérêt. La phase gaz dans l'enceinte de mesure à un volume $V_{gaz}$ ($V_{gaz} = V_{enceinte} - V_{matrice}$) et elle doit être en équilibre avec la matrice à analyser à une pression $P_{ini}$.
On impose ensuite une surpression initiale $P_0$ et on mesure le retour à l'équilibre à une nouvelle pression $P_{éq}$ (dû au transfert d'une partie du gaz vers la matrice à analyser). Dans ces conditions, en supposant que la température est constante et que l'équilibre thermodynamique peut être décrit par la loi de Henry, la constante de Henry est déterminée avec l'équation (1).

$$k_H = \frac{(P_0 - P_{éq}) \times V_{gaz} / R \times T}{V_{matrice}} \times \frac{1}{P_{éq} - P_{ini}} \qquad (1)$$

Où $P_0$ est la surpression imposée (Pa), $P_{éq}$ la pression à l'équilibre (Pa), $V_{gaz}$ la volume de la phase gaz (m$^3$), $V_{matrice}$ le volume de la matrice à analyser (m$^3$), R la constante des gaz parfait, T la température (K) et $P_{ini}$ la pression initiale à l'équilibre (Pa).

Matériel et méthode

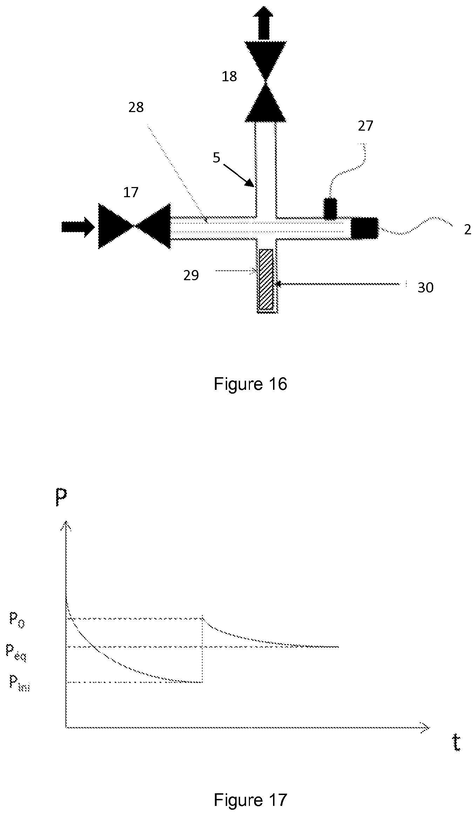

Un bloc de fromage, stocké plusieurs mois à 4°C puis plusieurs semaines à 19°C, a été utilisé pour faire la mesure. Un morceau de fromage de masse 0,33 g a été prélevé pour l'analyse. Comme la masse volumique du fromage est de 1120 kg.m$^{-3}$, le volume du morceau de fromage était de 0,3 cm$^3$. Le $CO_2$ provenait d'une bouteille de gaz (pureté >99,99%).
La mesure a été réalisée avec la sonde présentée sur la Figure 16. Elle est munie d'un capteur de pression (2) et d'un capteur pour mesurer la température (27) de la phase gazeuse à l'intérieur de la sonde. Deux vannes (une vanne (17) d'entrée et une vanne (18) de sortie) permettent le balayage de la sonde avec le gaz d'intérêt. Un tube interne (28) a été ajouté dans la sonde afin d'assurer que le balayage se fasse bien dans tout le volume de la sonde. La dernière partie de la sonde est constituée d'un tube cylindrique (29) dans lequel on a inséré le morceau de matrice (30) à analyser et qui a ensuite été fermé hermétiquement. La sonde est entièrement métallique et son volume à vide est de 1,3 cm$^3$.
Expérimentalement, la procédure suivante a été appliquée :

∘ On insère le morceau de matrice (30) à analyser (fromage ici) dans la sonde,
∘ On ferme la sonde,
∘ On balaie la sonde avec le gaz d'intérêt ($CO_2$ ici) pendant plusieurs secondes (en veillant à ce que la température reste constante et égale à la température ambiante),
∘ En fermant la vanne (18) de sortie du gaz, on impose une surpression dans la chambre gazeuse (5),
∘ On ferme la vanne (17) d'entrée du gaz. Il en résulte un transfert du gaz vers la matrice (30) à analyser, la pression diminue puis se stabilise à une valeur $P_{ini}$,
∘ De la même manière, on impose une seconde surpression $P_0$ et on attend la stabilisation de la pression à une valeur $P_{éq}$ supérieure à la première pression de stabilisation.

La Figure 17 présente l'allure globale de la variation de pression au cours de l'expérience pour déterminer la constante de Henry.

3. Résultats

Les pressions expérimentales suivantes ont été mesurées à la température de 18,5°C :

- $P_{ini}$=99,1 kPa
- $P_0$=126,4 kPa
- $P_{éq}$=119,2 kPa

La constante de Henry du $CO_2$ vis-à-vis du fromage déterminée avec l'équation (1) est dans ce cas égale à 5,0 x $10^{-4}$ mol.m$^{-3}$.Pa$^{-1}$. Cette valeur est du même ordre de grandeur que les valeurs de la littérature (Acerbi ([2]); Jakobsen ([8])). La différence avec la littérature peut venir de différences dans la composition du fromage ou de la durée d'affinage qui n'a pas été la même.

**Exemple 7 : détermination de la constante de Henry du $CO_2$ vis-à-vis de l'eau**

1. Théorie

L'équation (1) explicitée dans l'Exemple 6 a été aussi utilisée pour déterminer la constante de Henry du $CO_2$ dans l'eau.

2. Matériel et méthode

De l'eau distillée a été utilisée et le $CO_2$ provenait d'une bouteille de gaz (pureté >99,99%). Les expériences ont été réalisées dans une enceinte régulée en température à 18,5°C.

La mesure a été réalisée avec la sonde présentée sur la Figure 16 de l'Exemple 7. Elle est munie d'un capteur (2) de pression et d'un capteur (27) pour mesurer la température de la phase gazeuse à l'intérieur de la sonde. Deux vannes ((17), (18)) permettent le balayage de la sonde avec le gaz d'intérêt. Un tube interne (28) a été ajouté dans la sonde afin d'assurer que le balayage se fasse bien dans tout le volume de la sonde. La dernière partie de la sonde est constituée d'un tube cylindrique (29) dans lequel l'eau à analyser a été insérée et qui a ensuite été fermé hermétiquement. La sonde est entièrement métallique et son volume à vide est de 1,3 cm$^3$.

Expérimentalement, la procédure suivante a été appliquée :

- 1,0 mL d'eau est injecté dans la sonde
- On ferme la sonde
- On balaie la sonde avec le gaz d'intérêt ($CO_2$ ici) pendant plusieurs secondes
- En fermant la vanne (18) de sortie du gaz, on impose une surpression dans la chambre gazeuse (5).
- On ferme la vanne (17) d'entrée du gaz. Il en résulte un transfert du gaz vers l'eau, la pression diminue puis se stabilise à une valeur $P_{ini}$
- De la même manière, on impose une seconde surpression $P_0$ et on attend la stabilisation de la pression à une valeur $P_{éq}$ supérieure à la première pression de stabilisation.

3. Résultats

Les pressions expérimentales suivantes ont été mesurées à la température de 18,5°C :

- $P_{ini}$=99,6 kPa
- $P_0$=103,0 kPa
- $P_{éq}$=101,0 kPa

La constante de Henry du $CO_2$ vis-à-vis de l'eau déterminée avec l'équation (1) est égale à 3,5 x $10^{-4}$ mol.m$^{-3}$.Pa$^{-1}$. Cette valeur est très proche de celle de la littérature (Sander, 2015 ([13]); Versteeg ([14])) avec 8% d'écart et semble confirmer l'utilisation de la sonde pour la détermination de la constante de Henry.

**Exemple 8 : détermination de la viscosité d'un bitume et d'une pâte à pain**

1. Théorie

Le principe pour déterminer la viscosité d'une matrice est d'appliquer une surpression dans une phase gazeuse au contact de la matrice et de mesurer la variation de pression. En effet, la pression diminue au cours du temps due à l'augmentation de volume de la phase gazeuse. Dans ce cas on veille à choisir un gaz qui réagit ou se solubilise très peu dans la matrice ou les éléments utilisés pour assurer l'étanchéité sur la durée de la mesure, de sorte que toute la variation de pression soit attribuable à la variation de volume de la matrice.

Pour une géométrie cylindrique et en supposant que la variation de volume n'est due qu'à une variation de rayon du cylindre gazeux, la pression peut être reliée à la viscosité et à la déformation radiale avec l'équation (1).

$$P - P_{atm} = 2 \times \mu \times \frac{dR}{R} \times \frac{1}{dt} \qquad (1)$$

Où P est la pression dans la phase gaz en contact avec la matrice à analyser (Pa), $P_{atm}$ est la pression atmosphérique environnante au moment de l'essai (approximativement égale à 101,325 Pa au niveau de la mer), $\mu$ la viscosité de la matrice à analyser (Pa.s), R le rayon du cylindre gazeux créé dans la matrice à analyser (m) et t le temps (s). Idéalement, la pression initiale imposée doit être grande devant la variation de pression de l'atmosphère dans la matrice pendant la mesure.

La variation relative de rayon de la phase gazeuse $\frac{dR}{R}$ correspond à une variation de volume à un exposant 1/2 (car uniquement déformation radiale). En accord avec la loi des gaz parfaits (équation (2)), la variation relative de rayon de la phase gazeuse peut donc être déterminée avec la variation de pression dans la phase gaz avec l'équation (3).

$$P \times V = n \times R \times T \qquad (2)$$

Où V est le volume du cylindre gazeux dans la matrice à analyser ($m^3$), n la quantité de gaz (mol), R la constante des gaz parfaits ($J.mol^{-1}.K^{-1}$) et T la température (K).

$$\frac{\Delta R}{R} = \frac{P_1^{1/2} - P_2^{1/2}}{P_1^{1/2}} \qquad (3)$$

Où $P_1$ est la pression au temps $t_1$ (Pa) et $P_2$ la pression au temps $t_2 > t_1$ (Pa).

2. Matériel et methods

Les mesures de viscosité ont été réalisées sur le bitume commercial Azalt 70/100 (Total) et sur une pâte à pain de composition commerciale. Les mesures ont été réalisées avec de l'azote (pureté > 99,99%).

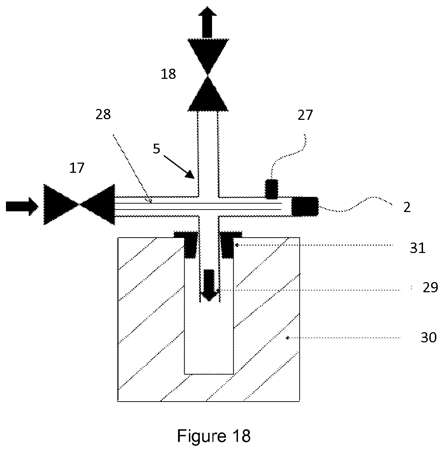

La sonde décrite sur la Figure 18 a été utilisée pour faire les mesures. Elle est munie d'un capteur de pression (2) et d'un capteur de temperature (27). Deux vannes (une vanne (17) d'entrée et une vanne (18) de sortie) permettent le balayage de la sonde avec le gaz d'intérêt. Un tube interne (28) a été ajouté dans la sonde afin d'assurer que le balayage se fasse bien dans tout le volume de la sonde. La quatrième partie de la sonde est constituée d'un tube cylindrique (29) ouvert en son extrémité qui est inséré dans la matrice à analyser (30) pour permettre au gaz d'arriver en contact avec la matrice. Un bouchon (31) permet d'assurer l'étanchéité avec la matrice à analyser (30). Les mesures ont été réalisées dans une chambre régulée en température à 22 $\pm$ 1°C pour le bitume et 19 $\pm$ 1°C pour la pâte à pain.

Expérimentalement, le protocole suivant a été appliqué :

- Une cavité cylindrique de hauteur 60 mm et de diamètre 7,5 mm est creusée dans la matrice à analyser (30) avec un foret,
- La sonde est insérée dans la cavité cylindrique comme illustré sur la Figure 18,
- Une surpression d'environ +2 kPa est imposée avec de l'azote en amenant du gaz par la vanne (17) d'entrée et en fermant la vanne (18) de sortie,
- La vanne (17) d'entrée du gaz est fermée et la pression est mesurée pendant plusieurs minutes avec une fréquence d'acquisition de 1 seconde.

3. Résultats

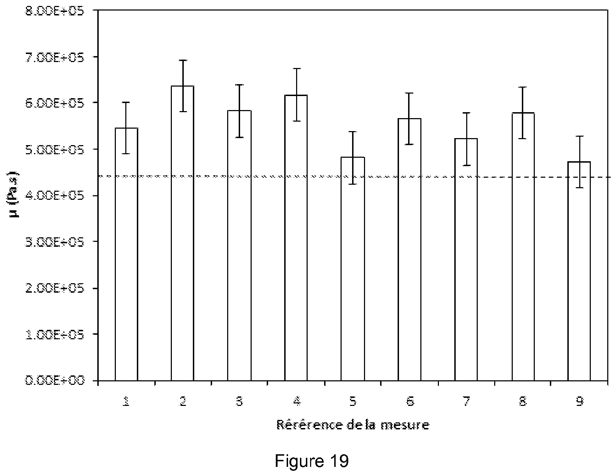

Avec le bitume, neuf essais ont été réalisés et les résultats sont regroupés sur la Figure 19. Les mesures sont reproductibles et la valeur moyenne de la viscosité est de 5,6 $\pm$ 0,6 x $10^5$ Pa.s. Cette valeur est du même ordre de grandeur que celle de la littérature (4,34 x $10^5$ Pa.s à 22°C (Mouazen, 2011 ([11])) et la différence peut provenir du léger écart de température (mesures réalisées avec la sonde à 21,4°C).

De la même manière avec la pâte à pain, une viscosité de 1,0 x 10$^5$ Pa.s a été déterminée à 19,5°C. Cette valeur est aussi en bon accord avec la littérature (Bloksma, 1975 ([10])).

**Exemple** 9 : **application de la sonde pour caractériser la pâte à pain à la manière d'un alvéographe**

Avec la sonde, il est possible de déterminer certaines caractéristiques d'une pâte à pain à la manière d'un alvéographe. Le principe, similaire à celui de l'alvéographe, est d'amener du gaz de façon continue au contact de la pâte à pain ce qui entraîne une augmentation de la pression et une déformation de la pâte à pain. La différence principale avec l'alvéographe vient du fait que les mesures avec la sonde se font à cœur dans un bloc de pâte (ce qui permet de conserver l'atmosphère réel de la pâte à pain).

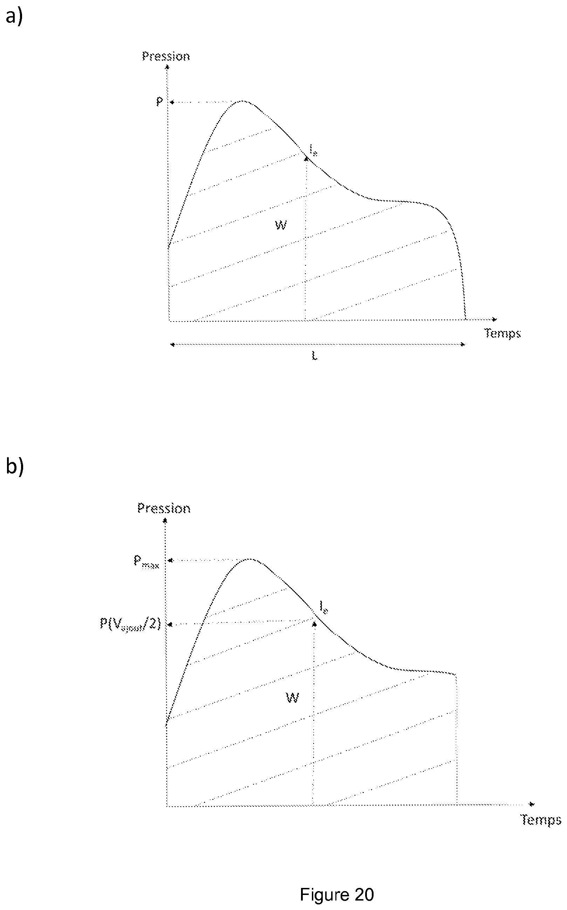

La Figure 20 présente l'allure d'une courbe de pression obtenue avec la sonde et la comparaison avec l'alvéographe. Sur la Figure 20-a correspondant à **l'alvéographe,** plusieurs données peuvent être obtenues pour caractériser la pâte à pain :

- La surpression maximale P caractérise la résistance à la déformation (ténacité de la pâte)
- L'aire sous la courbe W permet de caractériser la force de la farine, on parle de force boulangère
- L'index d'élasticité le caractérise la résistance élastique
- L'abscisse à la rupture L donne une information sur l'extensibilité de la pâte à pain

Avec la sonde (Figure 20-b), des informations similaires peuvent être obtenues (sauf l'abscisse à la rupture L qui n'est pas observée puisque la mesure se fait sur un gros bloc de pâte à pain et pas sur un film) :

- La surpression maximale $P_{max}$ caractérise la résistance à la déformation
- L'aire sous la courbe (après un temps de mesure pré-défini) W permet de caractériser la force de la farine
- L'index d'élasticité le caractérise la résistance élastique

Matériel et méthode

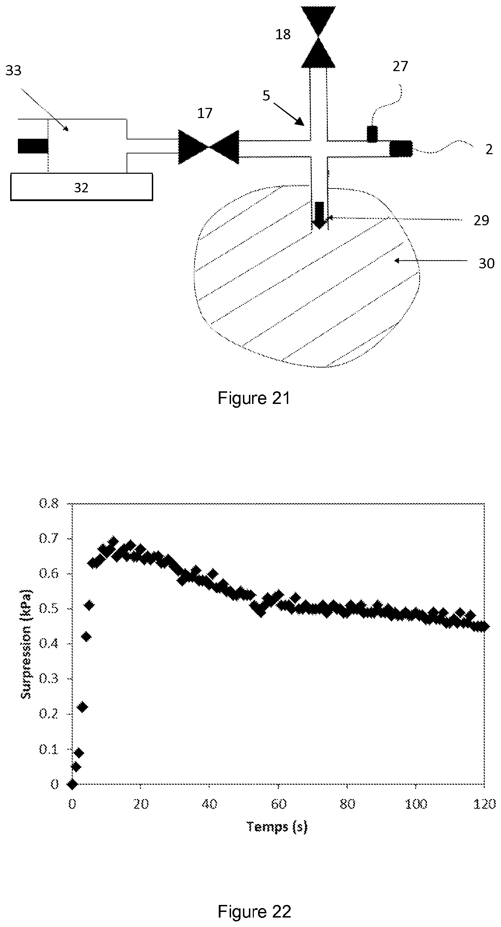

Les mesures ont été réalisées avec une pâte à pain de composition commerciale et avec de l'air. La sonde utilisée pour les mesures est décrite sur la Figure 21. Elle est munie d'un capteur de pression et d'un capteur de température. Deux vannes (une vanne (17) d'entrée et une vanne (18) de sortie) permettent l'entrée et la sortie du gaz. La sonde est aussi constituée d'un tube cylindrique (29) ouvert en son extrémité qui est inséré dans la matrice à analyser (30) pour permettre au gaz d'arriver en contact avec la matrice (30). Un pousse-seringue (32) et une seringue (33) permettent de contrôler l'arrivée en continu du gaz à un débit contrôlé.

La procédure expérimentale suivante a été adoptée:

◦ La sonde est plantée dans un bloc de pâte à pain
◦ Le gaz est injecté en continu avec l'aide du pousse-seringue au débit de 0,8 mL.min$^{-1}$ pendant 2 minutes
◦ La pression est mesurée pendant toute la durée de l'analyse avec une fréquence d'acquisition de 1 seconde. La température est aussi mesurée.

3. Résultats

La Figure 22 montre un exemple de signal obtenu avec la sonde pour caractériser la pâte à pain. La variation de pression est bien similaire à celle obtenue avec un alvéographe avec dans un premier temps une augmentation de pression qui caractérise la résistance à la déformation de la pâte ($P_{max}$=0,7 kPa) puis une diminution progressive de la pression qui traduit l'extensibilité de la pâte à pain.

En fonction de la pression maximale mesurée et de la diminution progressive de pression, la qualité de la pâte à pain peut ainsi être qualifiée.

**Exemple10 : détermination du coefficient de diffusion du CO$_2$ dans l'eau**

1. Théorie

Le principe de la mesure pour déterminer le coefficient de diffusion d'un gaz dissous dans une matrice alimentaire est d'appliquer une surpression dans une phase gazeuse (avec le gaz d'intérêt uniquement) en contact avec la matrice à analyser. Dû au transfert du gaz de la phase gazeuse vers la matrice (selon la loi de Henry) puis sa la diffusion dans la matrice (selon la loi de Fick), la pression diminue dans la phase gaz. L'évolution de cette diminution de pression peut être reliée aux propriétés de la matrice vis-à-vis du gaz d'intérêt, en particulier le coefficient de diffusion avec l'équation (1).

$$P(t) = \frac{C_0}{k_h} + \left(P_0 - \frac{C_0}{k_h}\right) \times \exp\left[\frac{t}{D_{CO2}} \times \left(\frac{R \times T \times D_{CO2} \times k_h}{e}\right)^2\right] \times \mathrm{erfc}\left[\sqrt{\frac{t}{D_{CO2}}} \times \left(\frac{R \times T \times D_{CO2} \times k_h}{e}\right)\right] \qquad (1)$$

Avec P la pression (Pa) au cours du temps t (s), $C_0$ la concentration initiale en gaz dissous dans la matrice à analyser ($mol.m^{-3}$), $k_h$ la constante de Henry ($mol.m^{-3}.Pa^{-1}$), $P_0$ la surpression initiale imposée (Pa), $D_{CO2}$ le coefficient de diffusion du gaz dans la matrice à analyser ($m^2.s^{-1}$) et e la hauteur de la phase gaz en contact avec la matrice à analyser (m).

2. Matériel et méthode

De l'eau distillée a été utilisée et le $CO_2$ provenait d'une bouteille de gaz (pureté >99,99%). Les expériences ont été réalisées à la température de 20,0°C.

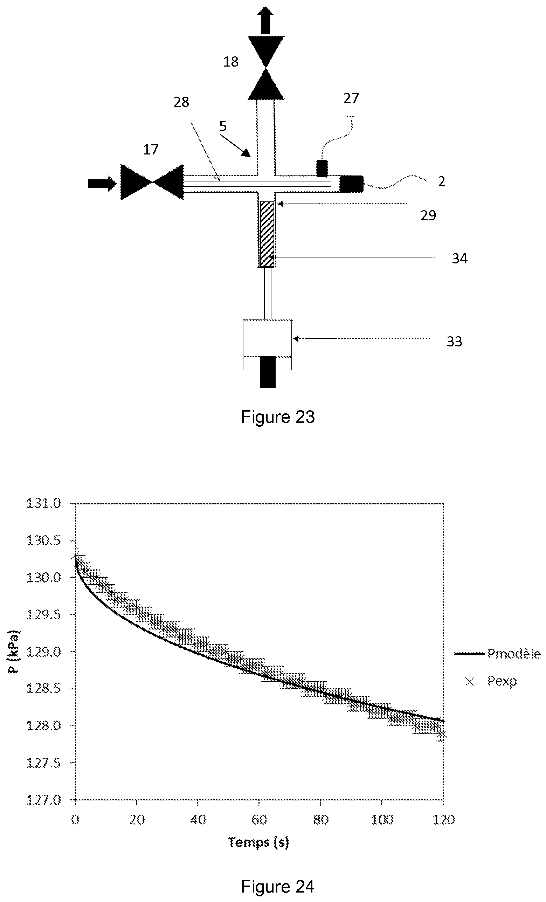

La mesure a été réalisée avec la sonde présentée sur la Figure 23. Elle est munie d'un capteur (2) de pression et d'un capteur (27) pour mesurer la température de la phase gazeuse à l'intérieur de la sonde. Deux vannes (une vanne d'entrée (17) et une vanne de sortie (18)) permettent le balayage de la sonde avec le gaz d'intérêt. Un tube interne (28) a été ajouté dans la sonde afin d'assurer que le balayage se fasse bien dans tout le volume de la sonde. La dernière partie de la sonde est constituée d'un tube cylindrique (29) dans lequel l'eau à analyser (34) a été insérée à l'aide d'une seringue (33) et qui a ensuite été fermé hermétiquement. La sonde est entièrement métallique et son volume à vide est de 1,3 cm$^3$.

Expérimentalement, la procédure suivante a été appliquée :

- Le balayage de la sonde est mis en fonctionnement en ouvrant les vannes d'entrée et de sortie du gaz.
- 1,0 mL d'eau est injecté dans la sonde avec la seringue
- Le balayage, avec l'eau dans la sonde, est maintenu pendant 1 minute
- On ferme la vanne de sortie du gaz
- En fermant la vanne de sortie du gaz, on impose une surpression $P_0$ dans la chambre gazeuse (5).
- On coupe l'arrivée en gaz en fermant la vanne d'entrée du gaz et on démarre la mesure de la pression pendant 120 secondes.

Le coefficient de diffusion du $CO_2$ dans l'eau a été évalué en considérant que la constante de Henry du $CO_2$ dans l'eau est égale à $3,4 \times 10^{-4}$ $mol.m^{-3}.Pa^{-1}$ et en faisant l'hypothèse que la surface de l'eau est initialement saturée en $CO_2$ dû au balayage en $CO_2$ pendant 1 minute avant le début de la mesure.

3. Résultats

La Figure 24 montre l'accord entre les valeurs de pressions expérimentales et celles déterminées avec le modèle (équation (5)) pour un coefficient de diffusion de $1,6 \times 10^{-9}$ $m^2.s^{-1}$. On constate que le modèle est en bon accord avec les valeurs expérimentales ce qui confirme que l'équation (5) décrit bien le transfert et la diffusion du $CO_2$ dans l'eau. Cette valeur du coefficient de diffusion du $CO_2$ dans l'eau est en bon accord avec les valeurs de la littérature avec 11% d'écart (Moultos et al., 2014 ([12]); Versteeg, 1988 ([14])).

Listes des références

1. Huc et al.: "Influence of salt content on eye growth in semi-hard cheese studied using magnetic resonance imaging and CO2 production measurements", International Dairy Journal (2014).

2. Acerbi et al.: "Impact of salt concentration, ripening temperature and ripening time on CO2 production of semi-hard cheese with propionic acid fermentation", Journal of Food Engineering, 177, 72-79 (2016).

3. Tveteraas O.: "A study of pressure decay in a closed CO2-water system", Master Thesis, 2011.

4. Ghaderi et al.: "Estimation of concentration-dependent diffusion coefficient in pressure-decay experiment of heavy oils and bitumen", Fluid phase equilibria, 2011.

5. Acerbi et al.: "An appraisal of the impact of compositional and ripening parameters on CO2 diffusivity in semi-hard cheese", Food Chemistry, 2016.

6. Chaix E. : « Caractérisation et modélisation des transferts de gaz (O2/CO2) dans le système emballage/aliment en lien avec les réactions de croissance microbienne (microbiologie prévisionnelle) », Thèse de l'université de Montpellier 2, 2014.

7. Chaix et al.: "Oxygen and carbon dioxide solubility and diffusivity in solid food matrix: a review of past and current knowledge", Comprehensive reviews in food science and food safety, Chaix et al, 2014.

8. Jakobsen M., Nygaard Jensen P. : « Assessment of carbon dioxide solubility coefficients for semi-hard cheeses : the effect of temperature and fat content », Eur. Food Res. Technol., 229, 287-294 (2009).

9. Grenier D., Laridon Y., Le Ray D., Challois S., Lucas T. : « Monitoring of single eye growth under known gas pressure: Magnetic resonance imaging measurements and insights into the mechanical behaviour of a semi-hard cheese », Journal of Food Engineering 171, 119-128, (2016).

10. Bloksma, A., Nieman, W., (1975). The effect of temperature on some rheological properties of wheat flour doughs. Journal of Texture studies 6(3), 343-361.

11. Mouazen, M., (2011). Evolution des propriétés rhéologiques des enrobés bitume, vers une loi vieillissement/-viscosité. École Nationale Supérieure des Mines de Paris.

12. Moultos, O.A., Tsimpanogiannis, I.N., Panagiotopoulos, A.Z., Economou, I.G., (2014). Atomistic molecular dynamics simulations of CO2 diffusivity in H2O for a wide range of temperatures and pressures. The Journal of Physical Chemistry B 118(20), 5532-5541.

13. Sander, R., (2015). Compilation of Henry's law constants (version 4.0) for water as solvent. Atmospheric Chemistry and Physics 15(8), 4399-4981.

14. Versteeg, G.F., Van Swaaij, W.P., (1988). Solubility and diffusivity of acid gases (carbon dioxide, nitrous oxide) in aqueous alkanolamine solutions. Journal of Chemical & Engineering Data 33(1), 29-34.

## Revendications

1. Dispositif de mesure de propriétés physico-chimiques vis-à-vis de gaz au contact d'un matériau, comprenant :

   - une extrémité supérieure (1), dans laquelle est inséré hermétiquement un capteur de pression (2) relié à un appareil (3) d'enregistrement et éventuellement de traitement d'un signal,
   - une extrémité inférieure (4), qui est en communication avec ledit capteur de pression (2) et qui est ouverte pour permettre l'insertion du dispositif de mesure dans ledit matériau et la formation d'une chambre gazeuse (5) entre ledit capteur de pression (2) et ledit matériau lorsque ledit dispositif de mesure est inséré dans celui-ci, **caractérisé en ce que** ledit dispositif comprend en outre :

      - un système (6) de balayage en un gaz, - au moins un moyen (10) d'entrée dudit gaz dans le dispositif, ledit moyen (10) étant relié à une source externe de gaz et n'étant pas un moyen de prélèvement du gaz inclus dans le matériau, et au moins un moyen (11) de sortie dudit gaz hors du dispositif,

   et que ledit dispositif est en une matière n'absorbant pas ledit gaz.

2. Dispositif selon la revendication 1, dans lequel ladite matière n'absorbant pas ledit gaz est choisie parmi le métal, le verre et des matériaux polymères préalablement saturés en ledit gaz ou traités pour ne pas absorber ledit gaz.

3. Dispositif selon l'une quelconque des revendications 1 ou 2, ledit dispositif étant un tube creux, éventuellement cylindrique.

4. Dispositif selon l'une quelconque des revendications précédentes, dans lequel la hauteur (7) dudit dispositif est supérieure ou égale à 5 mm, et la hauteur (8) entre l'extrémité inférieure (4) du dispositif et le capteur de pression (2) est supérieure à 1 mm.

5. Dispositif selon l'une quelconque des revendications précédentes, comprenant en outre au moins un moyen (9) de maintien en position du dispositif par rapport audit matériau.

6. Dispositif selon l'une quelconque des revendications précédentes, comprenant en outre une extension (12) reliée de manière étanche avec l'extrémité inférieure (4) dudit dispositif.

7. Dispositif selon l'une quelconque des revendications précédentes, dans lequel ledit matériau est une matrice alimentaire, notamment choisie parmi un produit fromager, un produit de boulangerie, une viande, un poisson, un produit à base de viande ou de poisson, un fruit, un légume, un produit à base de fruit ou de légume, une pâte alimentaire, et leurs mélanges, ou une matrice non alimentaire notamment choisie parmi du béton, du ciment, de l'asphalte, du plâtre, des polymères, des gels, de la terre, du bois, du silicone, du charbon, des roches, et leurs mélanges.

8. Procédé de mesure de la pression d'un gaz au contact d'un matériau, à l'aide d'un dispositif de mesure tel que défini dans l'une quelconque des revendications 1 à 7, comprenant les étapes suivantes :

   (a) insertion dudit dispositif de mesure dans le matériau,
   (b) éventuellement balayage en un gaz de la chambre gazeuse (5) au moyen du système (6) de balayage en un gaz, à pression constante,
   (c) augmentation ou diminution de la pression de la chambre gazeuse (5) au moyen du système (6) de balayage en un gaz jusqu'à une pression désirée, et
   (d) mesure de la pression et éventuellement de la température de la chambre gazeuse (5).

9. Procédé selon la revendication 8, dans lequel ledit gaz est choisi parmi le dioxyde de carbone, l'azote, l'oxygène, les gaz rares, les composés organiques volatiles, l'ammoniac, et un mélange de ceux-ci.

10. Procédé selon la revendication 8 ou 9, dans lequel l'augmentation de pression de l'étape (c) est réalisée progressivement jusqu'à fracturer ledit matériau.

11. Utilisation d'un dispositif de mesure tel que défini dans l'une quelconque des revendications 1 à 7, pour la mesure, dans un matériau, d'au moins une propriété physico-chimiques choisies parmi des propriétés de transport de matière vis-à-vis de gaz, en particulier le coefficient de diffusion, la constante d'équilibre gaz gazeux/gaz dissout, la concentration en gaz dissous et/ou la vitesse de production, et des propriétés mécaniques, en particulier l'élasticité, la viscosité, la visco-élasticité et le point de fracture.

12. Utilisation d'un dispositif de mesure tel que défini dans l'une quelconque des revendications 1 à 7, dans la préparation ou le suivi des caractéristiques de matériaux dans lesquelles un gaz est susceptible de se solubiliser et de diffuser.


**Patentansprüche**

1. Vorrichtung zum Messen physikalisch-chemischer Eigenschaften eines Materials in Bezug auf Gase, umfassend:

   - in oberes Ende (1), in das ein Drucksensor (2) hermetisch eingesetzt ist, der mit einer Vorrichtung (3) zum Aufzeichnen und gegebenenfalls Verarbeiten eines Signals verbunden ist,
   - ein unteres Ende (4), das mit dem Drucksensor (2) in Verbindung steht und offen ist, um das Einführen der Messvorrichtung in das Material und die Bildung einer Gaskammer (5) zwischen dem Drucksensor (2) und dem Material zu ermöglichen, wenn die Messvorrichtung in dieses eingeführt wird,
   **dadurch gekennzeichnet, dass** die Vorrichtung ferner umfasst:

   - ein Gas-Spülsystem (6),
   - mindestens ein Mittel (10) zum Einleiten des Gases in die Vorrichtung, wobei das Mittel (10) mit einer externen Gasquelle verbunden ist und ausdrücklich kein Mittel zur Entnahme eines im Material enthaltenen Gases ist, und
   - mindestens ein Mittel (11) zum Ableiten des Gases aus der Vorrichtung,

wobei die Vorrichtung aus einem Material hergestellt ist, das das Gas nicht absorbiert.

2. Vorrichtung nach Anspruch 1, wobei das genannte Material, das das genannte Gas nicht absorbiert, aus Metall, Glas und polymeren Materialien ausgewählt ist, die zuvor mit dem genannten Gas gesättigt wurden oder so behandelt wurden, dass sie das genannte Gas nicht absorbieren.

3. Vorrichtung nach Ansprüch 1 oder 2, wobei die Vorrichtung ein hohles, möglicherweise zylindrisches Rohr ist.

4. Vorrichtung nach einem der vorhergehenden Ansprüche, wobei die Höhe (7) der genannten Vorrichtung größer oder gleich 5 mm ist und die Höhe (8) zwischen dem unteren Ende (4) der Vorrichtung und dem Drucksensor (2) größer als 1 mm ist.

5. Vorrichtung nach einem der vorhergehenden Ansprüche, die ferner mindestens ein Mittel (9) zum Halten der Vorrichtung in Position relativ zu dem genannten Material umfasst.

6. Vorrichtung nach einem der vorhergehenden Ansprüche, die ferner eine Erweiterung (12) umfasst, die in dichter Weise mit dem unteren Ende (4) der Vorrichtung verbunden ist.

7. Vorrichtung nach einem der vorhergehenden Ansprüche, wobei das Material eine Lebensmittelmatrix ist, insbesondere ausgewählt aus einem Käseprodukt, einem Backprodukt, Fleisch, Fisch, einem Produkt auf Fleisch- oder Fischbasis, Obst, Gemüse, Produkten auf Obst- oder Gemüsebasis, Lebensmittelpaste und Mischungen davon, oder eine Nicht-Lebensmittelmatrix, insbesondere ausgewählt aus Beton, Zement, Asphalt, Gips, Polymeren, Gelen, Erde, Holz, Silikon, Kohle, Gestein und Mischungen davon.

8. Verfahren zum Messen des Drucks eines Gases im Kontakt mit einem Material, unter Verwendung einer , wie sie in einem der Ansprüche 1 bis 7 definiert ist, umfassend die folgenden Schritte:

   (a) Einführen der Messvorrichtung in das Material,
   (b) gegebenenfalls Spülen der Gaskammer (5) mit einem Gas mittels des Gasspülsystems (6) bei konstantem Druck,
   (c) Erhöhen oder Verringern des Drucks der Gaskammer (5) mittels des Gasspülsystems (6) auf einen gewünschten Druck, und
   (d) Messen des Drucks und gegebenenfalls der Temperatur der Gaskammer (5).

9. Verfahren nach Anspruch 8, wobei das Gas aus Kohlendioxid, Stickstoff, Sauerstoff, Edelgasen, flüchtigen organischen Verbindungen, Ammoniak und einer Mischung davon ausgewählt ist.

10. Verfahren nach Anspruch 8 oder 9, wobei die Druckerhöhung in Schritt (c) schrittweise durchgeführt wird, bis das Material aufbricht.

11. Verwendung einer Messvorrichtung, wie sie in einem der Ansprüche 1 bis 7 definiert ist, zur Messung in einem Material mindestens einer physikochemischen Eigenschaft, ausgewählt aus stofftransportbezogenen Eigenschaften gegenüber Gasen, insbesondere dem Diffusionskoeffizienten, der Gleichgewichtskonstante Gasphase/Gelöstgas, der Konzentration an gelöstem Gas und/oder der Produktionsgeschwindigkeit, sowie mechanischen Eigenschaften, insbesondere der Elastizität, der Viskosität, der Viskoelastizität und dem Bruchpunkt.

12. Verwendung einer Messvorrichtung, wie sie in einem der Ansprüche 1 bis 7 definiert ist, bei der Vorbereitung oder der Überwachung der Eigenschaften von Materialien, in denen sich ein Gas lösen und diffundieren kann.

**Claims**

1. Device for measuring the physical and chemical properties of gases in contact with a material, comprising:

   - an upper end (1), into which a pressure sensor (2) connected to a device (3) for recording and, optionally, processing a signal is hermetically inserted,
   - a lower end (4), which is in communication with said pressure sensor (2) and which is open to allow the insertion of the measuring device into said material and the formation of a gas chamber (5) between said pressure sensor (2) and said material when said measuring device is inserted therein,
**characterized in that** said device further comprises:

- a gas scanning system (6), - at least one means (10) for introducing said gas into the device, said means (10) being connected to an external gas source and not being a means for sampling the gas included in the material, and at least one means (11) for removing said gas from the device ,

and said device is made of a material that does not absorb said gas.

2. Device according to claim 1, wherein said material that does not absorb said gas is selected from metal, glass, and polymeric materials that are pre-saturated with said gas or treated so as not to absorb said gas.

3. Device according to any of claims 1 or 2, said device being a hollow tube, possibly cylindrical.

4. Device according to any of the preceding claims, wherein the height (7) of said device is greater than or equal to 5 mm, and the height (8) between the lower end (4) of the device and the pressure sensor (2) is greater than 1 mm.

5. Device according to any of the preceding claims, further comprising at least one means (9) for holding the device in position relative to said material.

6. Device according to any of the preceding claims, further comprising an extension (12) connected in a sealed manner to the lower end (4) of said device.

7. Device according to any of the preceding claims, wherein said material is a food matrix, in particular selected from a cheese product, a bakery product, meat, fish, a meat or fish-based product, fruit, vegetables, fruit or vegetable-based products, food paste, and mixtures thereof, or a non-food matrix, in particular chosen from concrete, cement, asphalt, plaster, polymers, gels, earth, wood, silicone, coal, rocks, and mixtures thereof.

8. Method for measuring the pressure of a gas in contact with a material, using a measuring device as defined in any of claims 1 to 7, comprising the following steps:

(a) inserting said measuring device into the material,
(b) optionally a gas sweeping of the gas chamber (5) with a gas by means of the gas sweeping system (6) at constant pressure,
(c) increasing or decreasing the pressure of the gas chamber (5) by means of the gas sweeping system (6) to a desired pressure, and
(d) measuring the pressure and optionally the temperature of the gas chamber (5).

9. Method according to claim 8, wherein said gas is selected from carbon dioxide, nitrogen, oxygen, rare gases, volatile organic compounds, ammonia, and a mixture thereof.

10. Method according to claim 8 or 9, wherein the pressure increase in step (c) is carried out gradually until said material fractures.

11. Use of a measuring device as defined in any of claims 1 to 7 for measuring, in a material, at least one physicochemical property selected from material transport properties with respect to gas, in particular the diffusion coefficient, the gas/dissolved gas equilibrium constant, the concentration of dissolved gas and/or the rate of production, and mechanical properties, in particular elasticity, viscosity, viscoelasticity, and fracture point.

12. Use of a measuring device as defined in any of claims 1 to 7 in the preparation or monitoring of the characteristics of materials in which a gas is likely to dissolve and diffuse.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

a)

b)

Figure 20

Figure 21

Figure 22

Figure 23

Figure 24

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0422260 A

**Littérature non-brevet citée dans la description**

- **ACERBI et al.** Impact of salt concentration, ripening temperature and ripening time on CO2 production of semi-hard cheese with propionic acid fermentation. *Journal of Food Engineering*, 2016 (2)
- **JAKOBSEN M.** ; **NYGAARD JENSEN P.** Assessment of carbon dioxide solubility coefficients for semi-hard cheeses : the effect of temperature and fat content. *Eur. Food Res. Technol*, 2009, vol. 229, 287-294
- **CHAIX et al.** Oxygen and carbon dioxide solubility and diffusivity in solid food matrix: a review of past and current knowledge. *Comprehensive reviews in food science and food safety*, 2014 (7)
- **TVETERAAS O**. A study of pressure decay in a closed CO2-water system. *Master Thesis*, 2011
- **GHADERI et al.** Estimation of concentration-dependent diffusion coefficient in pressure-decay experiment of heavy oils and bitumen. *Fluid phase equilibria*, 2011
- **HUC et al.** Influence of salt content on eye growth in semi-hard cheese studied using magnetic resonance imaging and CO2 production measurements. *International Dairy Journal*, 2014
- **ACERBI et al.** Impact of salt concentration, ripening temperature and ripening time on CO2 production of semi-hard cheese with propionic acid fermentation. *Journal of Food Engineering*, 2016, vol. 177, 72-79
- **TVETERAAS O.** A study of pressure decay in a closed CO2-water system. *Master Thesis*, 2011
- **ACERBI et al.** An appraisal of the impact of compositional and ripening parameters on CO2 diffusivity in semi-hard cheese. *Food Chemistry*, 2016
- **CHAIX E.** Caractérisation et modélisation des transferts de gaz (O2/CO2) dans le système emballage/aliment en lien avec les réactions de croissance microbienne (microbiologie prévisionnelle). *Thèse de l'université de Montpellier*, 2014, vol. 2

- **CHAIX** ; **CHAIX et al.** Oxygen and carbon dioxide solubility and diffusivity in solid food matrix: a review of past and current knowledge. *Comprehensive reviews in food science and food safety*, 2014
- **JAKOBSEN M.** ; **NYGAARD JENSEN P.** Assessment of carbon dioxide solubility coefficients for semi-hard cheeses : the effect of temperature and fat content. *Eur. Food Res. Technol.*, 2009, vol. 229, 287-294
- **GRENIER D.** ; **LARIDON Y.** ; **LE RAY D.** ; **CHALLOIS S.** ; **LUCAS T.** Monitoring of single eye growth under known gas pressure: Magnetic resonance imaging measurements and insights into the mechanical behaviour of a semi-hard cheese. *Journal of Food Engineering*, 2016, vol. 171, 119-128
- **BLOKSMA, A.** ; **NIEMAN, W.** The effect of temperature on some rheological properties of wheat flour doughs. *Journal of Texture studies*, 1975, vol. 6 (3), 343-361
- **MOUAZEN, M.** Evolution des propriétés rhéologiques des enrobés bitume, vers une loi vieillissement/viscosité. *École Nationale Supérieure des Mines de Paris*, 2011
- **MOULTOS, O.A.** ; **TSIMPANOGIANNIS, I.N.** ; **PANAGIOTOPOULOS, A.Z.** ; **ECONOMOU, I.G.** Atomistic molecular dynamics simulations of CO2 diffusivity in H2O for a wide range of temperatures and pressures. *The Journal of Physical Chemistry B*, 2014, vol. 118 (20), 5532-5541
- **SANDER, R.** Compilation of Henry's law constants (version 4.0) for water as solvent. *Atmospheric Chemistry and Physics*, 2015, vol. 15 (8), 4399-4981
- **VERSTEEG, G.F.** ; **VAN SWAAIJ, W.P.** Solubility and diffusivity of acid gases (carbon dioxide, nitrous oxide) in aqueous alkanolamine solutions. *Journal of Chemical & Engineering Data*, 1988, vol. 33 (1), 29-34